(19) Europäisches Patentamt / European Patent Office / Office européen des brevets

(11) **EP 3 125 141 B1**

(12) **EUROPEAN PATENT SPECIFICATION**

(45) Date of publication and mention
of the grant of the patent:
**20.05.2020   Bulletin 2020/21**

(51) Int Cl.:
*A61N 1/362* *(2006.01)*      *A61N 1/368* *(2006.01)*

(21) Application number: **16179318.7**

(22) Date of filing: **13.07.2016**

(54) **SYSTEM, METHOD, AND PROGRAM FOR BIOMEDICAL SIMULATION**

SYSTEM, VERFAHREN UND PROGRAMM ZUR BIOMEDIZINISCHEN SIMULATION

SYSTÈME, PROCÉDÉ ET PROGRAMME DE SIMULATION BIOMÉDICALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2015   JP 2015151649**

(43) Date of publication of application:
**01.02.2017   Bulletin 2017/05**

(73) Proprietor: **FUJITSU LIMITED**
**211-8588 Kanagawa (JP)**

(72) Inventor: **KADOOKA, Yoshimasa**
**Kawasaki-shi, Kanagawa 211-8588 (JP)**

(74) Representative: **Haseltine Lake Kempner LLP**
**Lincoln House, 5th Floor**
**300 High Holborn**
**London WC1V 7JH (GB)**

(56) References cited:
WO-A2-2015/073927      US-A1- 2010 292 556
US-A1- 2013 197 881      US-A1- 2014 257 047
US-A1- 2015 042 646

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The embodiments discussed herein are related to a system, method, and program for biomedical simulation.

BACKGROUND

Abnormalities may develop in conduction pathways for cardiac excitation due to myocardial infarction, heart failure, or the like. When abnormalities occur in the conduction pathways, the contraction timing between the interventricular septum and the ventricular free walls goes out of synchronization, which results in decreased cardiac ejection fraction (EF) and systolic pressure. The ejection fraction is calculated by dividing the volume of blood pumped out by the heart during each heartbeat (stroke volume) by the volume of blood within the left ventricle in diastole. With the development of symptoms in a patient such as decreased ejection fraction, it is sometimes the case that the patient experiences difficulties in maintaining a normal life.

For such patients with serious cardiac disorders, a cardiac resynchronization therapy (CRT) device, which is a type of pacemaker, is implanted in the body based on applicable medical guidelines. The guidelines set out, for example, patients with an ejection fraction $\leq$ 35% and an electrocardiogram (ECG) QRS duration $\geq$ 130 milliseconds as targets for CRT implantation. The QRS duration is the time from the onset of the Q wave to the offset of the S wave in the QRS complex (representing ventricular activation) on an ECG.

A CRT device has three lead wires attached thereto and an electrode is provided on the end or in the middle of each lead wire. Two of the lead wires are implanted into the right atrium and right ventricle of a patient and the third lead wire is placed into the coronary sinus. Then, the CRT device applies potentials to the electrodes on the lead wires in such a manner as to cause the ventricles and the interventricular septum to contract in synchronization with cardiac pacing in the patient.

In the case with patients who respond to biventricular pacing by CRT device implantation, the cardiac motion is resynchronized so that the heart beats in a rhythmic and coordinated manner, resulting in improved cardiac pump function. On the other hand, yet approximately 30% of patients fail to respond to biventricular pacing by CRT device implantation (termed "non-responders"). In addition, doctors identify the optimal electrode locations through trial and error during operations, resulting in increased surgical time. This imposes substantial physical and psychological burdens on patients, especially non-responders. In view of such problems, there have been proposed various techniques associated with CRT application determination, for example, techniques for selecting patients suitable for CRT and techniques for identifying the optimal installation locations for electrodes of a pacemaker.

Japanese Laid-open Patent Publication No. 2011-98175

Japanese National Publication of International Patent Application No. 2008-534165

Japanese National Publication of International Patent Application No. 2010-528683

Jason Constantino, Yuxuan Hu, Natalia A. Trayanova, "A computational approach to understanding the cardiac electromechanical activation sequence in the normal and failing heart, with translation to the clinical practice of CRT", Progress in Biophysics and Molecular Biology, 2012 Oct-Nov, 110 372-379

Sermesant M, Chabiniok R, Chinchapatnam P, Mansi T, Billet F, Moireau P, Peyrat JM, Wong K, Relan J, Rhode K, Ginks M, Lambiase P, Delingette H, Sorine M, Rinaldi CA, Chapelle D, Razavi R, Ayache N., "Patient-specific electromechanical models of the heart for the prediction of pacing acute effects in CRT: A preliminary clinical validation" Medical Image Analysis, 2012 Jan; 16(1):201-215

Okada J, Sasaki T, Washio T, Yamashita H, Kariya T, Imai Y, Nakagawa M, Kadooka Y, Nagai R, Hisada T, Sugiura S., "Patient Specific Simulation of Body Surface ECG using the Finite Element Method" Pacing and Clinical Electrophysiology, 2013 Mar; 36(3):309-321

US 2013/0197881 A1 discloses a method and system for patient-specific planning of cardiac therpy, such as cardiac resynchronization therapy, based on preoperative clinical data and medical images, such as ECG data, MRI data and ultrasound data. A patient-specific computational heart model is generated, and used to simulate cardiac therapies such as CRT.

CRT devices are very expensive and CRT implantation is invasive. Therefore, including non-responders in eligible patients for CRT implantation leads to wasted medical expenses and increased insurance costs. In the United States, for example, medical treatments are particularly expensive and it is sometimes the case that patients are responsible for part of medical treatment costs even if they have health insurance. If patients turn out to be non-responders after CRT implantation, doctors are at risk for lawsuits seeking enormous amounts of money in damages, brought by the patients or insurance firms forced to incur the medical expenses. A doctor on the losing end of a lawsuit has to bear the medical treatment costs. Thus, in CRT, a plurality of parties, including patients, doctors, and insurance firms, assume a risk to pay the costs.

On the other hand, running heart simulation based on an accurate heart model of a patient himself/herself allows accurate estimation of the optimal electrode locations and the effectiveness of CRT before performing CRT implantation. The accurate estimation of the CRT effectiveness contributes to reducing the incidents of CRT implantation in non-responders, which results in a reduction in medical expenses.

Appropriate judgments made by the parties bearing the risk to pay medical expenses eliminate performing ineffective treatments. However, there is no conventional system that allows the risk-bearing parties to share results of heart simulation and reflects the results in decision making to deliver appropriate treatment. As a result, CRT is used to treat even patients in whom CRT is not expected to be effective, thus increasing medical expenses.

Other than CRT, there are therapeutic methodologies capable of predicting prognosis of a patient by biomedical simulation using a model reproducing organs of the patient. The problem of increased medical expenses due to the practice of ineffective treatments attributed to non-existence of appropriate sharing of results obtained from biomedical simulation is not unique to CRT, but is common to other medical treatments.

SUMMARY

It is desirable to reduce medical expenses. According to embodiments of aspects of the invention, there are provided biomedical simulation systems, biomedical simulation methods and computer programs which, when executed by a biomedical simulation system, causes the biomedical simulation system to perform a biomedical simulation method, all as defined in the claims. The claims define the scope of the invention.

BRIEF DESCRIPTION OF DRAWINGS

The invention is described, by way of example only, with reference to the following drawings, in which:

FIG. 1 illustrates a configuration example of a biomedical simulation system according to a first embodiment;
FIG. 2 illustrates an example of CRT device implantation;
FIG. 3 illustrates an example of a system configuration according to a second embodiment;
FIG. 4 is a flowchart illustrating a procedure for determining whether to provide CRT by the use of CRT simulation;
FIG. 5 illustrates an example of a hardware configuration of a hospital information system;
FIG. 6 is a block diagram illustrating functions of individual systems;
FIG. 7 illustrates an example of procedures for CRT treatment and medical expense reimbursement according to the second embodiment;
FIG. 8 is a flowchart illustrating an example of a processing procedure for determining dynamic system simulation parameters;
FIG. 9 is a flowchart illustrating an example of a procedure for heart simulation;
FIG. 10 is a flowchart illustrating an example of a processing procedure for determining electrical system simulation parameters;
FIG. 11 is a flowchart illustrating an example of a processing procedure for parameter tuning;
FIG. 12 is a flowchart illustrating an example of a processing procedure for calculating electrical potentials;
FIG. 13 is a flowchart illustrating an example of a processing procedure of heart simulation for each CRT device;
FIG. 14 illustrates an example of a procedure of heart simulation designed for a biventricular pacing CRT device;
FIG. 15 illustrates an example of a procedure of heart simulation designed for a triventricular pacing CRT device;
FIG. 16 illustrates an example of a procedure of heart simulation designed for a quadriventricular pacing CRT device;
FIG. 17 is a flowchart illustrating an example of a processing procedure for determining optimal CRT device and electrode locations based on various indexes;
FIG. 18 illustrates an example of a procedure of CRT treatment and medical expense reimbursement according to a third embodiment;
FIG. 19 illustrates an example of a processing procedure according to a fourth embodiment;
FIG. 20 illustrates an example of a procedure of CRT treatment and medical expense reimbursement according to the fourth embodiment;
FIG. 21 illustrates an example of a procedure of CRT treatment and medical expense reimbursement according to a fifth embodiment;
FIG. 22 illustrates an example of a procedure of CRT treatment and medical expense reimbursement according to a sixth embodiment;
FIG. 23 illustrates an example of a procedure of CRT treatment and medical expense reimbursement according to a seventh embodiment;
FIG. 24 illustrates an example of a procedure of CRT treatment and medical expense reimbursement according to an eighth embodiment;

FIG. 25 illustrates an example of a procedure of CRT treatment and medical expense reimbursement according to a ninth embodiment;

FIG. 26 illustrates an example of a procedure of CRT treatment and medical expense reimbursement according to a tenth embodiment;

FIG. 27 illustrates an example of a processing procedure according to an eleventh embodiment;

FIG. 28 illustrates an example of a procedure of CRT treatment and medical expense reimbursement according to the eleventh embodiment;

FIG. 29 illustrates an example of a processing procedure according to a twelfth embodiment;

FIG. 30 illustrates an example of a processing procedure according to a thirteenth embodiment;

FIG. 31 illustrates an example of a processing procedure according to a fourteenth embodiment;

FIG. 32 illustrates an example of a processing procedure according to a fifteenth embodiment; and

FIG. 33 illustrates an example of a processing procedure according to a sixteenth embodiment.

DESCRIPTION OF EMBODIMENTS

Several embodiments will be described below with reference to the accompanying drawings, wherein like reference numerals refer to like elements throughout. Note that two or more of the embodiments below may be combined for implementation in such a way that no contradiction arises.

(a) First Embodiment

Next described is a first embodiment. The first embodiment is directed to a technique for investigating the effectiveness of an operation by running biomedical simulation using a biomedical model of a patient's organ and reimbursing the expenses of the operation by insurance only when the operation is expect to produce an improvement in the patient, to thereby prevent unnecessary treatments and reduce medical expenses.

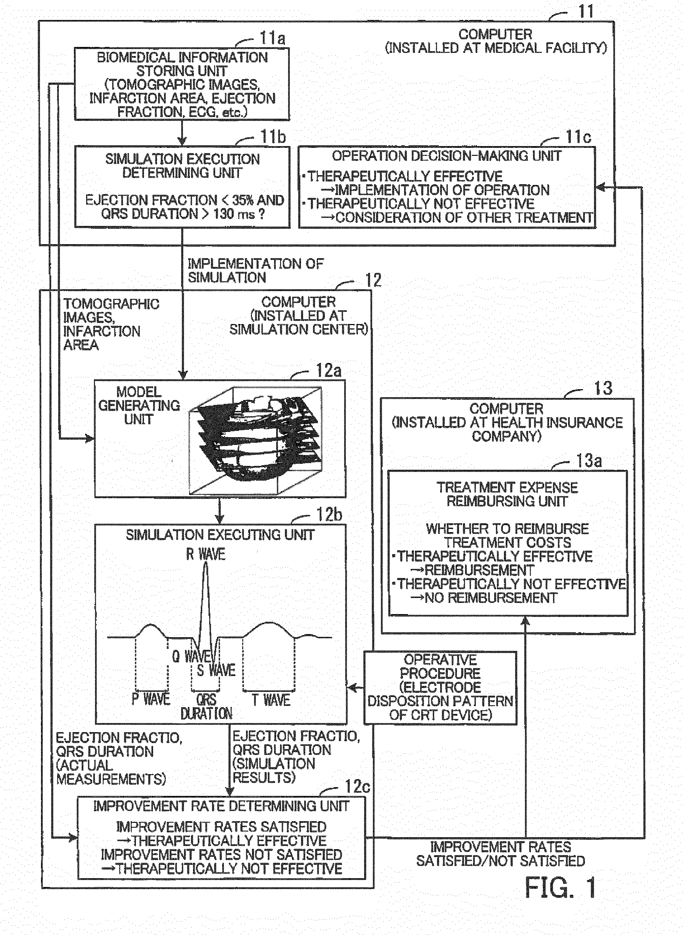

FIG. 1 illustrates a configuration example of a biomedical simulation system according to the first embodiment. The biomedical simulation system of FIG. 1 includes a plurality of computers 11 to 13. The computer 11 is installed at, for example, a medical facility for performing an operation on a patient concerned. The computer 12 is installed at, for example, a simulation center for running biomedical simulation. The computer 13 is installed at, for example, a health insurance company for reimbursing medical treatment costs.

The computer 11 includes a biomedical information storing unit 11a, a simulation execution determining unit 11b, and an operation decision-making unit 11c. The biomedical information storing unit 11a stores therein biomedical information specific to the patient. The biomedical information includes tomographic images of a particular organ (e.g. heart) of the patient, information on an area of infarction in the heart, the ejection fraction, and ECG data. The simulation execution determining unit 11b determines, based on the biomedical information, whether to run biomedical simulation. For example, in the case where the operation to be performed on the patient is CRT implantation, the simulation execution determining unit 11b determines to run biomedical simulation if the ejection fraction falls below a predetermined value (for example, 35%) and the QRS duration exceeds a predetermined value (e.g. 130 milliseconds). The determination result of whether to perform biomedical simulation is transmitted to the computer 12. The operation decision-making unit 11c decides, when an improvement rate determining unit 12c of the computer 12 has determined that results obtained from the biomedical simulation meet a predetermined improvement rate, to perform the operation on the patient. Following the decision, the operation is carried out by a doctor.

The computer 12 includes a model generating unit 12a, a simulation executing unit 12b, and the improvement rate determining unit 12c. The model generating unit 12a generates a biomedical model of the particular organ of the patient based on the biomedical information when the simulation execution determining unit 11b has determined to perform biomedical simulation. For example, the model generating unit 12a generates a stereoscopic model of the heart (heart model) of the patient based on the tomographic images of the patient's heart. In addition, if the heart of the patient has an area of infarction, the model generating unit 12a sets the area of infarction to be represented at a corresponding location in the heart model.

Based on the biomedical model generated by the model generating unit 12a, the simulation executing unit 12b runs biomedical simulation of post-treatment conditions of the patient's particular organ. In the case where the simulation is related to implantation of a CRT device, an electrode disposition pattern of the CRT device, for example, is input to the simulation executing unit 12b as an operative procedure. Then, the simulation executing unit 12b runs the simulation to predict post-operative heartbeat conditions following an operation of disposing electrodes of the CRT device based on the input electrode disposition pattern. The heart simulation outputs, for example, ECG data and an ejection fraction value as its simulation results.

The improvement rate determining unit 12c determines whether results of the biomedical simulation run by the simulation executing unit 12b meet a predetermined improvement rate associated with symptoms. For example, in the case where heart simulation has been performed, results of the simulation are compared to actual measurements taken from the patient with respect to ECG QRS duration and ejection fraction. If the simulation predicts an improvement in each of the QRS duration and the ejection fraction at a predetermined improvement rate or more, treatment using the operative procedure adopted by the simulation executing unit 12b is considered to be effective. On the other hand, neither the QRS duration nor the ejection fraction shows an improvement at the predetermined improvement rate or more, the treatment using the adopted operative procedure is considered to provide no benefit. The determination result of whether the simulation results satisfy the predetermined improvement rate is transmitted to other computers 11 and 13.

The computer 13 includes a treatment expense reimbursing unit 13a. When the improvement rate determining unit 12c has determined that the results of the biomedical simulation satisfy the predetermined improvement rate, the treatment expense reimbursing unit 13a determines to reimburse treatment costs for the operation of the patient. On the other hand, when the improvement rate determining unit 12c has determined that the biomedical simulation results fail to satisfy the predetermined improvement rate, the treatment expense reimbursing unit 13a determines not to reimburse the treatment costs for the operation.

According to the above-described biomedical simulation system, biomedical simulation is determined to be run for a patient whose symptoms have become so worse that the patient needs an operation, and the biomedical simulation then predicts the patient's post-operative organ conditions following the operation. The execution of the biomedical simulation of the patient's organ enables an accurate understanding about the effectiveness of the operation. As a result, the operation is carried out only when it is expected to produce an improvement in the patient, and the expenses of the operation are then reimbursed by insurance. Thus, according to the first embodiment, an operation is carried out only when biomedical simulation predicts that the operation will yield a treatment effect, which prevents the practice of ineffective operations and thus enables a reduction in medical expenses.

Note that each component of the individual computers 11 to 13 may be incorporated in a computer different from one illustrated in FIG. 1. For example, the operation decision-making unit 11c may be incorporated in the computer 13. In addition, the improvement rate determining unit 12c may be incorporated in the computer 13. Further, both the operation decision-making unit 11c and the improvement rate determining unit 12c may be incorporated in the computer 13.

The operation decision-making unit 11c may be configured by separate first and second decision-making units. In this case, when the improvement rate determining unit 12c has determined that the results of the biomedical simulation satisfy the predetermined improvement rate, for example, the first decision-making unit outputs an application for insurance reimbursement. When the improvement rate determining unit 12c has determined that the results of the biomedical simulation satisfy the predetermined improvement rate and, then, the first decision-making unit has output the insurance reimbursement application, the second decision-making unit decides to perform the operation on the patient. In this regard, the first decision-making unit and the second decision-making unit are incorporated in, for example, the computers 11 and 13, respectively. Herewith, when insurance reimbursement is sought, it is possible to check the validity of the insurance reimbursement application.

In addition, the operation decision-making unit 11c may decide to perform the operation on the patient when confirming that the improvement rate determining unit 12c has determined that the results of the biomedical simulation satisfy the predetermined improvement rate and the treatment expense reimbursing unit 13a has determined to reimburse the medical treatment costs of the patient. This prevents a situation where it turns out after the operation that no insurance reimbursement is available.

The treatment expense reimbursing unit 13a may determine to reimburse the treatment costs for the operation and costs for running the biomedical simulation when the operation has been performed on the patient, and may determine to reimburse the costs for running the biomedical simulation when the operation is not performed. Herewith, the patient is able to receive insurance reimbursement for the biomedical simulation service even when no reimbursement is made for operation costs.

A device characteristics data storing unit may further be provided in the biomedical simulation system to store device characteristics data indicating characteristics of devices possibly used in the operation of the patient. In this case, the simulation executing unit 12b may refer to the device characteristics data and run the simulation in which characteristics of each device are reflected. Herewith, it is possible to improve the accuracy of the biomedical simulation and also appropriately evaluate the suitability of each of the devices to the patient.

Further, with respect to each of a plurality of operative procedures applicable to the patient, the simulation executing unit 12b is able to run biomedical simulation to predict the patient's post-operative conditions following an operation using the operative procedure. In this case, the improvement rate determining unit 12c selects, amongst the applicable operative procedures, one with simulation results showing the highest improvement rate, and determines whether the improvement rate satisfies the predetermined improvement rate. This allows a proposal for the best suited operative procedure. For example, by running simulation for each of a plurality of electrode disposition patterns associated with CRT device implementation, the optimal electrode disposition pattern is determined. Then, the improvement rate determining unit 12c transmits information indicating the operative procedure with the highest improvement rate to an information processor installed at the medical facility for performing the operation on the patient. This enables presentation of the optimal operative procedure (e.g. the optimal electrode disposition pattern associated with CRT device implantation) to a doctor who will operate on the patient. As a result, the surgical time is reduced, alleviating the physical burden on the patient.

In the case where there are a plurality of electrode disposition patterns each yielding an improvement rate equal to or more than the predetermined improvement rate, the improvement rate determining unit 12c may transmit these electrode disposition patterns to the information processor at the medical facility. For example, the improvement rate determining unit 12c transmits electrode disposition patterns with the top three improvement rates or so. This allows the doctor to select, amongst the plurality of electrode disposition patterns, an appropriate disposition pattern and apply it to the operation. For example, in an actual operation, the condition of the patient may not allow the installation of the electrodes at locations indicated by the optimal electrode disposition pattern. In such a case, the doctor installs the electrodes according to, not the optimal electrode disposition pattern, but an electrode disposition pattern with the second or third highest improvement rate. Herewith, the certainty of the CRT implantation is increased.

The simulation executing unit 12b may run biomedical simulation for each of a plurality of devices available for the patient's operation. In this case, the improvement rate determining unit 12c selects, amongst the available devices, one with simulation results showing the highest improvement rate, and determines whether the improvement rate satisfies the predetermined improvement rate. Herewith, it is possible to appropriately predict the treatment effectiveness produced when the device best suited to the patient is used. In addition, the improvement rate determining unit 12c may transmit information on the device yielding the highest improvement rate to the computer 11 installed at the medical facility for performing the operation on the patient. This enables presentation of the optimal device for the patient to the doctor. In addition, the improvement rate determining unit 12c may transmit the information on the device yielding the highest improvement rate also to the computer 13 installed at the health insurance company for reimbursing medical treatment costs. This allows the health insurance company to select an appropriate device in view of the cost-effectiveness.

Note that each of the simulation execution determining unit 11b, the operation decision-making unit 11c, the model generating unit 12a, the simulation executing unit 12b, the improvement rate determining unit 12c, and the treatment expense reimbursing unit 13a may be implemented, for example, by a processor of the computer with which the unit is incorporated. In addition, the biomedical information storing unit 11a may be implemented, for example, by memory or a storage device of the computer 11. In FIG. 1, each line connecting the individual components represents a part of communication paths, and communication paths other than those illustrated in FIG. 1 are also configurable.

(b) Second Embodiment

Next described is a second embodiment. The second embodiment is directed to a computer system providing support for determining whether to implement treatment involving CRT device implantation. The treatment involving CRT device implantation is hereinafter simply referred to as "CRT treatment".

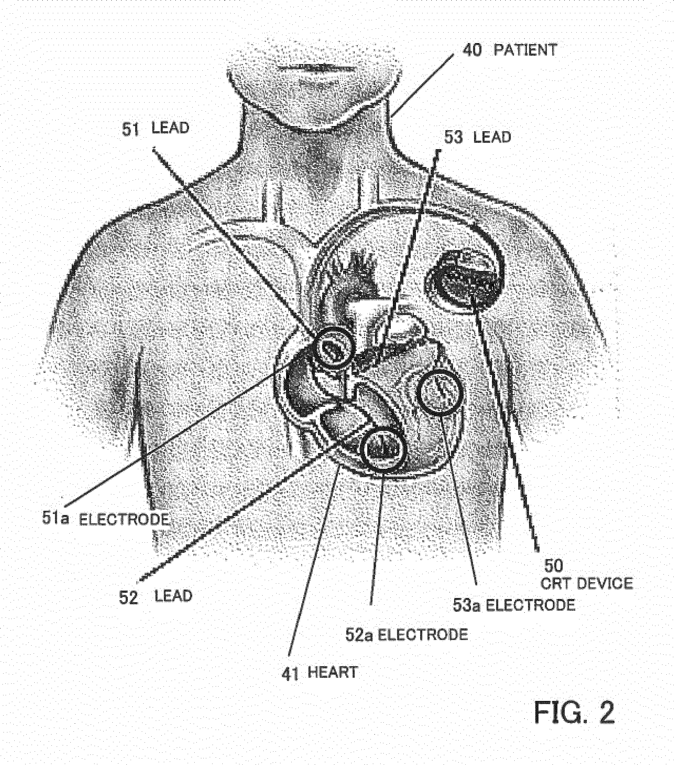

FIG. 2 illustrates an example of CRT device implantation. In CRT, a CRT device 50, which is a type of pacemaker, is implanted in a patient 40 with cardiac disorders. The CRT device 50 has a plurality of leads 51 to 53, and electrodes 51a, 52a, and 53a are provided on the individual leads 51 to 53, respectively. The leads 51 to 53 are inserted into the body of the patient 40, and the electrodes 51a, 52a, and 53a are placed at appropriate locations in a heart 41. For example, the electrode 51a on the lead 51 is implanted into the right atrium; the electrode 52a on the lead 52 is implanted into the right ventricle; and the electrode 53a on the lead 53 is placed into the coronary sinus. The CRT device 50 applies potentials to the individual electrodes 51a, 52a, and 53a in such a manner as to cause the ventricles and the interventricular septum to contract in synchronization with pacing of the heart 41 of the patient 40.

For example, an area of infarction of the patient 40 with myocardial infarction does not conduct electric current and is therefore unable to conduct the electrical signals of the impulse conduction system normally. The CRT device 50 plays a role in supporting the impulse conduction. Note that the conditions of the impulse conduction of the heart 41 of the patient 40 before CRT treatment differ depending on the location of the infarction. Therefore, the optimal disposition locations of the electrodes 51a, 52a, and 53a of the CRT device 50 depend on the patient 40. CRT simulation is able to identify a CRT device and electrode disposition pattern suitable for the patient 40.

The computer system according to the second embodiment uses non-invasive CRT simulation to predict, prior to CRT treatment, the effects (improvements in the ECG QRS duration and ejection fraction) following the implantation of a CRT device. Then, based on prediction results, the system selects a CRT device produced by a manufacturer best suited for the patient 40, and presents usage instructions for the CRT device (such as the optimal electrode installation locations and electrode usage configuration (such as biventricular pacing and triventricular pacing). Then, the system according to the second embodiment allows information based on the prediction results to be shared among the doctor, the patient, and the healthcare insurance company.

Herewith, the best-suited manufacturer's CRT device and the optimal electrode installation locations are identified in advance and the surgical time is therefore reduced, which in turn alleviates the burden on the patient. If the patient is likely to be a non-responder, a different therapeutic approach is selected to thereby reduce unnecessary physical and financial burden of the patient and also prevent wasted medical expenses. In addition, by predicting the effects following the CRT implantation and sharing information on the prediction results with the patient, it is possible to reduce the risk of the doctor being sued in a medical malpractice lawsuit.

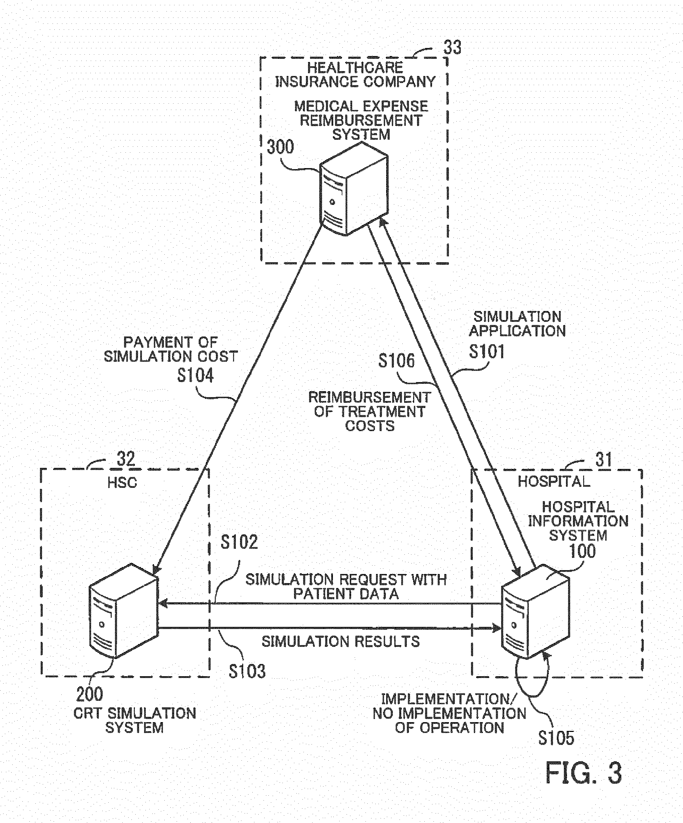

FIG. 3 illustrates an example of a system configuration according to the second embodiment. A hospital information system 100 is installed at a hospital 31. The hospital information system 100 is a computer system for managing medical charts and biomedical information of patients. A CRT simulation system 200 is installed at a heart simulation center (HSC) 32 for providing CRT simulation services. The CRT simulation system 200 is a computer system for running heart simulation associated with implantation of a CRT device (CRT simulation). A medical expense reimbursement system 300 is installed at a healthcare insurance company 33. The medical expense reimbursement system 300 is a computer system for performing procedures to reimburse the hospital 31 for costs involved in treatment covered by insurance.

The hospital information system 100, the CRT simulation system 200, and the medical expense reimbursement system 300 of FIG. 3 cooperate to execute CRT simulation smoothly and allow results obtained from the simulation to be shared among the doctor, the patient, and the healthcare insurance company 33. Specifically, when, at the hospital 31, the doctor has determined CRT treatment to be appropriate for the patient, a simulation application is sent from the hospital information system 100 to the medical expense reimbursement system 300 (step S101). After receiving approval from the healthcare insurance company 33, the hospital information system 100 sends, to the CRT simulation system 200, a simulation request to which the hospital 31 has attached data of the patient needed for simulation (step S102). The patient data includes biomedical data to be used to create a heart model specific to the patient.

At the HSC 32, the optimal electrode installation locations of a CRT device in the patient are predicted using a simulator, and results obtained from the simulation are sent to the hospital information system 100 (step S103). The simulation results include a combination pattern of electrode locations predicted to produce the greatest improvement in the cardiac function of the patient as well as data of the cardiac function (such as ECG data, ejection fraction, dP/dt, and data visualizing ventricular motion) associated with each of all the combination patterns. In this regard, the HSC 32 makes a claim for cost incurred in the simulation against the healthcare insurance company 33. In response, the medical expense reimbursement system 300 processes procedures to pay the simulation cost and then informs the CRT simulation system 200 of the procedure result (step S104). The payment procedures involve, for example, sending money to cover the simulation cost to a bank account of the HSC 32.

At the hospital 31, the hospital information system 100 determines whether to provide CRT treatment, based on the simulation results sent by the HSC 32 (step S105). When the hospital information system 100 has determined to provide CRT treatment, the doctor carries out CRT implantation. Following the CRT implantation, the hospital 31 sends a request for reimbursement of the medical treatment costs to the healthcare insurance company 33. In response, the medical expense reimbursement system 300 processes procedures to pay the CRT treatment costs and then informs the hospital information system 100 of the procedure result (step S106). The payment procedures involve, for example, sending money to cover the CRT treatment costs to a bank account of the hospital 31. The effective use of the CRT simulation results in the above-described manner reduces unproductive treatments. Note however that, on the patient's own free will, he/she is able to still undergo CRT treatment by paying all the expenses.

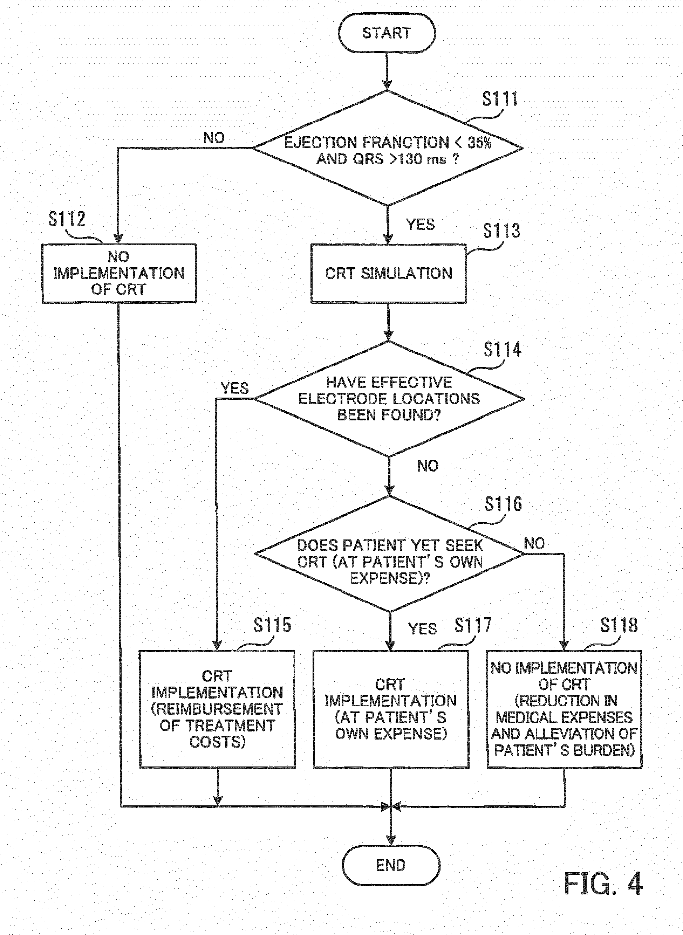

FIG. 4 is a flowchart illustrating a procedure for determining whether to provide CRT by the use of CRT simulation.

[Step S111] The hospital information system 100 determines, based on the biomedical information of the patient, whether the following conditions are satisfied: the ejection fraction is below 35%; and the QRS duration exceeds 130 milliseconds. In addition, the heart failure being refractory to medical therapy may be added as a further condition. If the conditions are satisfied, the procedure moves to step S113. If not, the procedure moves to step S112.

[Step S112] Since the state of the patient is not too critical to undergo CRT treatment, the hospital information system 100 determines not to provide CRT to the patient and ends the procedure.

[Step S113] The hospital information system 100 requests the CRT simulation system 200 to run CRT simulation. In response to the request, the CRT simulation system 200 runs CRT simulation.

[Step S114] Based on results obtained from the simulation, the CRT simulation system 200 determines whether electrode locations having positive treatment effects have been found. If such electrode locations have been found, the procedure moves to step S115. If not, the procedure moves to step S116.

[Step S115] The CRT simulation system 200 sends information on the optimal electrode locations having positive treatment effects to the hospital information system 100. Then, the doctor at the hospital 31 carries out CRT treatment according to the optimal electrode locations obtained as the CRT simulation results. In this case, the CRT simulation results are also sent to the medical expense reimbursement system 300 and the medical treatment costs are reimbursed by the healthcare insurance company 33, and subsequently the procedure ends.

[Step S116] The CRT simulation system 200 informs the hospital information system 100 of no detection of electrode locations having positive treatment effects. At the hospital 31, the doctor asks the patient or a person acting on behalf of the patient whether the patient seeks CRT treatment at his/her own expense. If the patient decides to undergo CRT treatment at his/her own expense, the procedure moves to step S117. If not, the procedure moves to step S118.

[Step S117] The doctor carries out CRT treatment. In this case, the medical treatment costs are borne by the patient. Subsequently, the procedure ends.

[Step S118] The doctor determines to provide no CRT treatment and try a different therapeutic approach. This achieves a reduction in medical expenses by not providing ineffective CRT treatment, and also alleviates the burden on the patient.

The use of CRT simulation in the above-described manner allows the doctor to insert lead wires at the optimal electrode installation locations from the start of the operation based on the simulation results. As a result, the surgical time is reduced. In addition, the use of CRT simulation helps to make clear not only the effectiveness of CRT treatment but also which party will incur the medical treatment costs, thus preventing incidents of being involuntarily burdened with the medical treatment costs. For example, in the case of not using the CRT simulation, the CRT treatment is implemented regardless of whether the patient is a non-responder as long as the conditions (the ejection fraction < 35%, and the QRS duration > 130 milliseconds) defined in the guidelines are met. In the case of using the CRT simulation, on the other hand, the CRT simulation is implemented prior to CRT implantation and, only when electrode installation locations predicted to have positive treatment effects are identified, CRT implantation is carried out. This allows the healthcare insurance company 33 to reimburse the medical treatment costs of the CRT treatment only when the promise of producing a certain degree of effect by the CRT implementation is objectively determined, and thus avoid incurring costs for ineffective medical treatments.

In addition, highly accurate determination of the effectiveness of the CRT implementation prevents the incidents of patients turning out to be non-responders after surgery, which reduces the risk of doctors being sued in lawsuits seeking damages and the like. Further, even if a patient who has decided not to undergo CRT implantation falls critically ill later, the risk of the doctor being sued for not implementing CRT is reduced because not undergoing CRT implantation was a choice made by the patient or a person acting on behalf of the patient. Additionally, even if electrode installation locations predicted to have positive treatment effects are not identified on a patient, the patient still has the option to undergo CRT implantation by paying all the expenses. In this case, the patient receives CRT treatment after being clear about he/she being likely to be a non-responder, and therefore the doctor is free from being sued for damages by the patient.

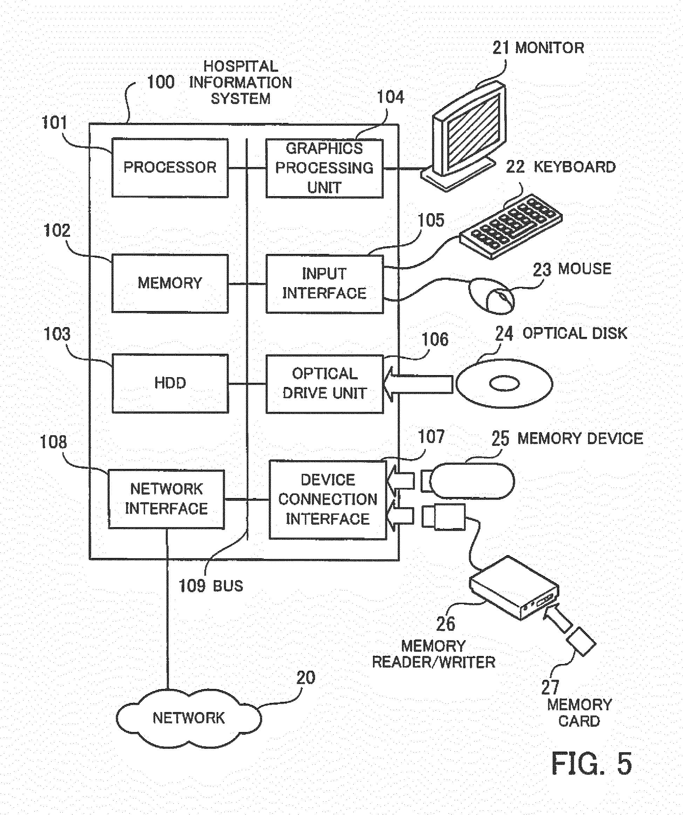

Next the second embodiment is described in greater detail. First, an account is given for a hardware configuration of the system according to the second embodiment. FIG. 5 illustrates an example of a hardware configuration of the hospital information system. Overall control of the hospital information system 100 is exercised by a processor 101. To the processor 101, memory 102 and a plurality of peripherals are connected via a bus 109. The processor 101 may be a multi-processor. The processor 101 is, for example, a central processing unit (CPU), a micro processing unit (MPU), a graphics processing unit (GPU), or a digital signal processor (DSP). At least part of the functions implemented by executing a program by the processor 101 may be implemented as an electronic circuit, such as an application specific integrated circuit (ASIC) and a programmable logic device (PLD). The memory 102 is used as a main memory device of the hospital information system 100. The memory 102 temporarily stores at least part of an operating system (OS) program and application programs to be executed by the processor 101. The memory 102 also stores therein various types of data to be used by the processor 101 for its processing. As the memory 102, a volatile semiconductor storage device such as random access memory (RAM) may be used.

The peripherals connected to the bus 109 include a hard disk drive (HDD) 103, a graphics processing unit 104, an input interface 105, an optical drive unit 106, a device connection interface 107, and a network interface 108. The HDD 103 magnetically writes and reads data to and from a built-in disk, and is used as a secondary storage device of the hospital information system 100. The HDD 103 stores therein the OS program, application programs, and various types of data. Note that a non-volatile semiconductor storage device (solid state drive, or SSD) such as flash memory may be used as a secondary storage device in place of the HDD 103.

To the graphics processing unit 104, a monitor 21 is connected. According to an instruction from the processor 101, the graphics processing unit 104 displays an image on a screen of the monitor 21. A cathode ray tube (CRT) display or a liquid crystal display, for example, may be used as the monitor 21. To the input interface 105, a keyboard 22 and a mouse 23 are connected. The input interface 105 transmits signals sent from the keyboard 22 and the mouse 23 to the processor 101. Note that the mouse 23 is just an example of pointing devices, and a different pointing device such as a touch panel, a tablet, a touch-pad, and a track ball, may be used instead. The optical drive unit 106 reads data recorded on an optical disk 24 using, for example, laser light. The optical disk 24 is a portable storage medium on which data is recorded in such a manner as to be read by reflection of light. Examples of the optical disk 24 include a digital versatile disc (DVD), a DVD-RAM, a compact disk read only memory (CD-ROM), a CD recordable (CD-R), and a CD-rewritable (CD-RW).

The device connection interface 107 is a communication interface for connecting peripherals to the hospital information system 100. To the device connection interface 107, for example, a memory device 25 and a memory reader/writer 26 may be connected. The memory device 25 is a storage medium having a function for communicating with the device connection interface 107. The memory reader/writer 26 is a device for writing and reading data to and from a memory card 27 which is a card type storage medium. The network interface 108 is connected to a network 20. Via the network 20, the network interface 108 transmits and receives data to and from other computers and communication devices.

The hardware configuration described above achieves the processing functions of the second embodiment. Note that the CRT simulation system 200 and the medical expense reimbursement system 300 may be built with the same hardware configuration as the hospital information system 100. In addition, each of the computers 11 to 13 of the first embodiment may also be built with the same hardware configuration as that of the hospital information system 100 of FIG. 5.

The hospital information system 100 achieves its processing functions according to the second embodiment, for example, by executing a program stored in a computerreadable storage medium. The program describing processing content to be implemented by the hospital information system 100 may be stored in various types of storage media. For example, the program to be executed by the hospital information system 100 may be stored in the HDD 103. The processor 101 loads at least part of the program stored in the HDD 103 into the memory 102 and then runs the program. In addition, the program to be executed by the hospital information system 100 may be stored in a portable storage medium, such as the optical disk 24, the memory device 25, and the memory card 27. The program stored in the portable storage medium becomes executable after being installed on the HDD 103, for example, under the control of the processor 101. Alternatively, the processor 101 may run the program by directly reading it from the portable storage medium.

According to the example of FIG. 5, a single computer forms the computer system; however, the computer system may be a parallel processing system made up of a plurality of computers. For example, if the CRT simulation system 200 is configured as a parallel processing system with a plurality of computers, it is possible to run highly accurate CRT simulation in a short amount of time.

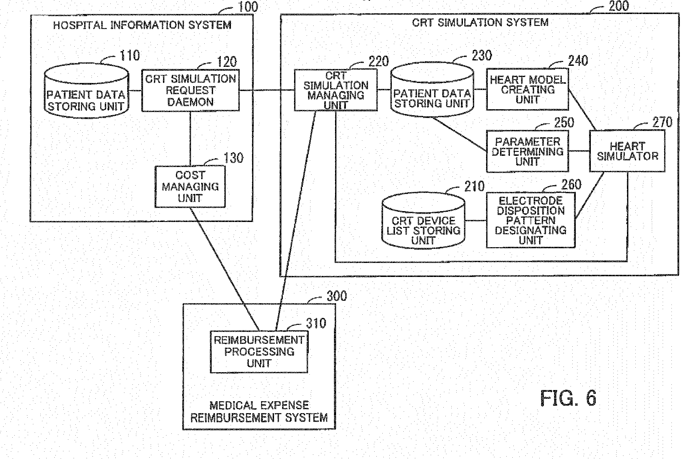

Next described are functions of the individual systems to implement the second embodiment. FIG. 6 is a block diagram illustrating functions of individual systems. The hospital information system 100 includes a patient data storing unit 110, a CRT simulation request daemon 120, and a cost managing unit 130. The patient data storing unit 110 stores therein image data obtained by a picture archiving and communication system (PACS) and an electronic health record for each patient. The PACS is a system for managing medical images. For example, images produced by magnetic resonance imaging (MRI) and computed tomography (CT) are stored in the patient data storing unit 110 via the PACS.

The CRT simulation request daemon 120 makes a request to the CRT simulation system 200 for CRT simulation, and acquires simulation results obtained by the CRT simulation. In addition, based on the simulation results, the CRT simulation request daemon 120 determines whether to implement CRT or not. The cost managing unit 130 manages medical treatment costs. For example, the cost managing unit 130 receives notice of whether insurance coverage is available from the medical expense reimbursement system 300. Then, if CRT implantation covered by insurance is carried out, the cost managing unit 130 makes a claim for the operative cost to the medical expense reimbursement system 300.

The CRT simulation system 200 includes a CRT device list storing unit 210, a CRT simulation managing unit 220, a patient data storing unit 230, a heart model creating unit 240, a parameter determining unit 250, an electrode disposition pattern designating unit 260, and a heart simulator 270. The CRT device list storing unit 210 is a list of a plurality of CRT devices produced and distributed by different manufacturers. For example, the CRT device list storing unit 210 includes, for each of the CRT devices, the name of its manufacturer, the name of its model, and the number of electrodes of the device. The CRT simulation managing unit 220 manages execution of CRT simulation. For example, upon receiving a CRT simulation request, the CRT simulation managing unit 220 stores patient data attached to the CRT simulation request in the patient data storing unit 230. Then, the CRT simulation managing unit 220 issues predetermined execution instructions to the heart model creating unit 240, the parameter determining unit 250, and the heart simulator 270 to thereby implement CRT simulation.

The patient data storing unit 230 stores therein data specific to each patient subject to CRT simulation. The data stored in the patient data storing unit 230 is similar to that stored in the patient data storing unit 110 of the hospital information system 100. The heart model creating unit 240 creates a heart model of a patient based on data of the patient stored in the patient data storing unit 230. The heart model creating unit 240 creates the patient's heart model using, for example, tomographic images of MRI. The parameter determining unit 250 determines values of parameters used in CRT simulation, according to the patient's heart. For example, the parameter determining unit 250 adjusts the parameter values in such a manner that ECG data obtained by motion simulation on the patient's heart model without CRT implementation coincides with actual ECG data of the patient.

The electrode disposition pattern designating unit 260 sets, for each CRT device, a plurality of electrode disposition patterns on the patient's heart model created by the heart model creating unit 240. The heart simulator 270 runs, with respect to each CRT device, CRT simulation for the case of the CRT device being implanted in the patient. The CRT simulation is run based on product information of the targeted CRT device. If a plurality of electrode disposition patterns have been proposed for a single CRT device, the heart simulator 270 runs CRT simulation for all the disposition patterns. The results of the repeatedly executed CRT simulation are sent to the CRT simulation managing unit 220 and then used to determine the optimal CRT device and electrode locations for the patient.

The medical expense reimbursement system 300 includes a reimbursement processing unit 310. The reimbursement processing unit 310 determines whether to reimburse expenses incurred in the CRT treatment, based on the CRT simulation results. Then, when an operation covered by insurance is carried out, the reimbursement processing unit 310 arranges for payment of the costs.

In FIG. 6, each line connecting the individual components represents a part of communication paths, and communication paths other than those illustrated in FIG. 6 are also configurable. Further, the function of each component illustrated in FIG. 6 is implemented, for example, by causing a computer to execute a program module corresponding to the component.

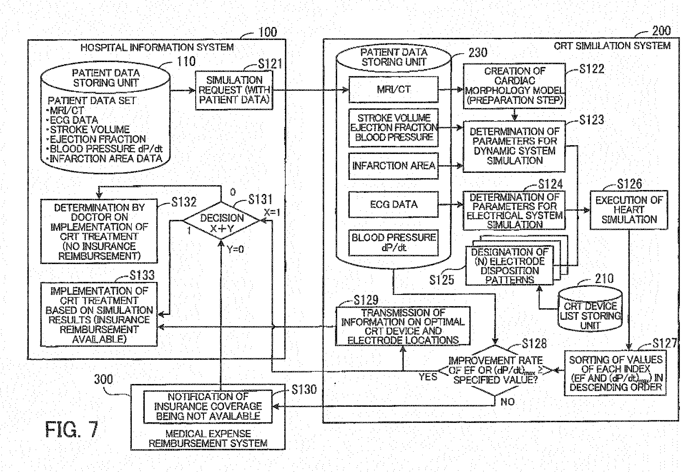

Next the CRT treatment and medical expense reimbursement processes according to the second embodiment are described in detail. FIG. 7 illustrates an example of procedures for the CRT treatment and medical expense reimbursement according to the second embodiment. The patient data storing unit 110 of the hospital information system 100 stores patient data to be used for CRT simulation. The patient data includes, for example, tomographic image data of MRI or CT, 12-lead ECG data, stroke volume, left ventricular ejection fraction, change in blood pressure (dP/dt), and infarction area data. The stroke volume is the amount of blood ejected to the aorta with each contraction of the heart. The stroke volume is measured in milliliter (ml), for example. The stroke volume is calculated by the following equation:

$$SV = EDV - ESV;$$

where SV is the stroke volume, EDV is the end diastolic volume, and ESV is the end systolic volume. The change in blood pressure is the rate of change in blood pressure in the left ventricle. The change in blood pressure is a commonly used index to evaluate the contractile function of the entire heart and measured by cardiac catheterization, Doppler echocardiography, or the like. The infarction area data indicates the location of an infarction area of the patient's heart.

The CRT simulation request daemon 120 of the hospital information system 100 determines whether the patient is a target of the CRT treatment. For example, the CRT simulation request daemon 120 acquires the ejection fraction and ECG data of the patient from the patient data storing unit 110 and calculates the QRS duration from the ECG data. Then, if the ejection fraction is below a predetermined value (e.g. 35%) and the QRS duration exceeds a predetermined value (e.g. 130 milliseconds), the CRT simulation request daemon 120 determines that the patient is a target of the CRT treatment. When having determined the patient as a CRT treatment target, the CRT simulation request daemon 120 sends a simulation request to the CRT simulation system 200 (step S121). The patient data of the patient is attached to the simulation request. The CRT simulation request daemon 120 may send the simulation request with the patient data in encrypted form.

In the CRT simulation system 200, the CRT simulation managing unit 220 stores the received patient data in the patient data storing unit 230. Subsequently, based on the tomographic images of the patient's heart, the heart model creating unit 240 creates morphology data representing the heart structure of the patient (such as a cardiac morphology model and a thorax (chest) model) and simulation mesh data (such as a finite element mesh model) (step S122). In addition, the heart model creating unit 240 performs segmentation of the thorax (chest) and sets electrical conductivities (for individual organs, fat, muscle, and bone), and also performs segmentation of the heart and sets an electrical conductivity of the heart. The infarction area is set to have non-excitable tissues. Further, the heart model creating unit 240 maps data indicating the cardiac fiber direction on the cardiac morphology model. This model creating process is a preparation step for CRT simulation.

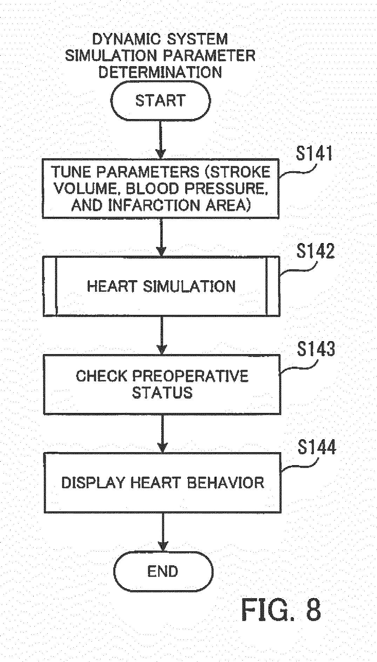

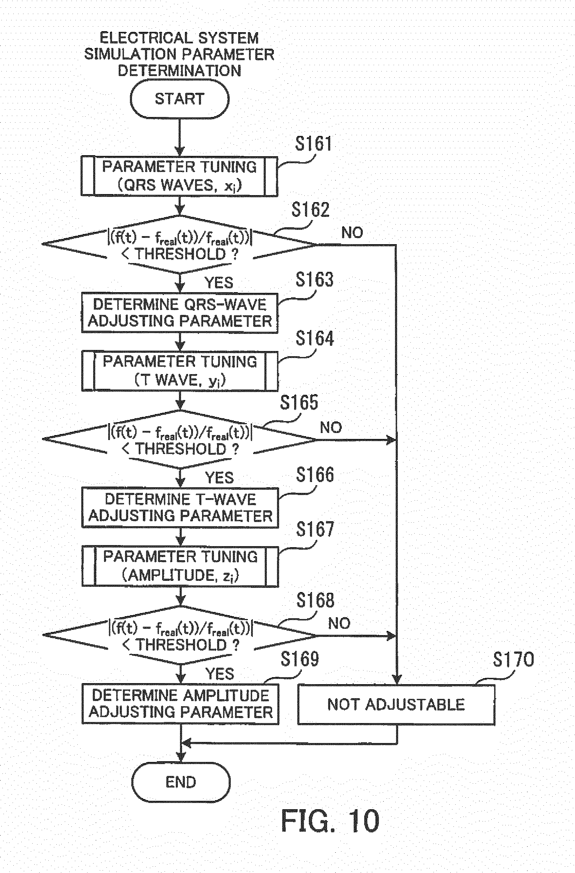

Next, the parameter determining unit 250 determines values of parameters used in cardiac dynamic system simulation based on the stroke volume, blood pressure, and infarction area data (step S123). Details of the parameter determining process for the dynamic system simulation are described later (see FIG. 8). Further, the parameter determining unit 250 determines values of parameters used in cardiac electrical system simulation based on the ECG data (step S124). This allows the heart of the patient to be reconstructed in the CRT simulation system. Details of the parameter determining process for the electrical system simulation are described later (see FIG. 10).

The electrode disposition pattern designating unit 260 acquires the list of CRT devices from the CRT device list storing unit 210. Then, using electrode information included in the acquired CRT device list, the electrode disposition pattern designating unit 260 designates, for each CRT device, a plurality of electrode disposition patterns (N patterns, where N is an integer greater than or equal to 1), each of which has different combination of locations for individual electrodes implanted in the right ventricle and on the outside of the left ventricle (step S125). Note that each of the electrode disposition patterns indicates the name of a corresponding CRT device and electrode locations for implantation of the CRT device. The heart simulator 270 runs simulation of the heart behavior for each electrode disposition pattern of each CRT device (step S126).

By running the simulation, estimated ejection fraction, estimated $(dP/dt)_{max}$, and the like are output as simulation results for each electrode disposition pattern. $(dP/dt)_{max}$ is the maximum value among values of dP/dt (the time differential of ventricular pressure) changing during the contraction cycle. The CRT simulation managing unit 220 associates each of the indexes (e.g. the ejection fraction and $(dP/dt)_{max}$) calculated as the simulation results with the corresponding electrode disposition pattern used for the calculation. Then, the CRT simulation managing unit 220 sorts values of each of the indexes (the ejection fraction and $(dP/dt)_{max}$) calculated by the repeated heart simulation in descending order (step S127).

Once completing the heart simulation for all the electrode disposition patterns of all the CRT devices, the CRT simulation managing unit 220 identifies a CRT device and its electrode disposition pattern yielding the highest improvement rate. For example, the CRT simulation managing unit 220 compares the ejection fraction and $(dP/dt)_{max}$ of the patient before CRT implantation against those obtained as the simulation results, and calculates the improvement rate for each electrode disposition pattern. Then, the CRT simulation managing unit 220 determines an electrode disposition pattern having the highest improvement rate. Note that a CRT device and electrode locations corresponding to the electrode disposition pattern having the highest improvement rate are identified as the optimal CRT device and electrode locations.

The CRT simulation managing unit 220 determines whether the improvement rate of the optimal CRT device and electrode locations is greater than or equal to a specified value (step S128). For example, when the ejection fraction is predicted to improve by 10% or more or the maximum value of (dP/dt) is predicted to improve by 100 Pascal per second (Pa/s) or more, the CRT simulation managing unit 220 determines that the improvement rate is greater than or equal to the specified value. If the improvement rate is greater than or equal to the specified value, the CRT simulation managing unit 220 sends information on the optimal CRT device and electrode locations to the hospital information system 100 (step S129). In the hospital information system 100, the cost managing unit 130 acquires the information on the optimal CRT device and electrode locations. Upon acquiring the optimal CRT device and electrode locations, the cost managing unit 130 plugs in 1 for a decision variable X (the initial value is 0).

If the improvement rate is below the specified value, the CRT simulation managing unit 220 notifies the medical expense reimbursement system 300 of the patient being predicted not to experience an improvement (i.e., the patient being likely to be a non-responder). In the medical expense reimbursement system 300, the reimbursement processing unit 310 receives the notice from the CRT simulation system 200, and then determines that CRT treatment for the patient is not covered by insurance and records the determination. Subsequently, the reimbursement processing unit 310 notifies the hospital information system 100 of insurance coverage for the CRT treatment of the patient being unavailable (step S130).

Upon receiving the notice of insurance coverage being unavailable, the cost managing unit 130 of the hospital information system 100 plugs in 0 for a decision variable Y (the initial value is 0). Then, the cost managing unit 130 calculates the value obtained by X + Y (step S131). When X + Y equals 0, the cost managing unit 130 urges the doctor to determine whether to perform CRT implantation to the patient. For example, the cost managing unit 130 displays, on a terminal device used by the doctor, information indicating that the patient is likely to be a non-responder and insurance coverage for the CRT implantation will not be available. The doctor speaks with the patient in reference to the result of the decision (X + Y), and then determines whether to perform CRT implantation on the patient (step S132). In this case, if CRT implantation is determined to be performed, no reimbursement for the medical expenses will be made by the healthcare insurance company 33. When X + Y equals 1, the cost managing unit 130 displays, on the terminal device of the doctor, information indicating that CRT treatment is expected to be effective in the patient and will be covered by insurance. Based on the simulation results, the doctor carries out CRT implantation (step S133). In this case, the medical treatment costs will be reimbursed by the healthcare insurance company 33.

In the above-described manner, the CRT simulation results are shared among the doctor, the patient, and the healthcare insurance company 33, to thereby prevent unnecessary CRT treatments. In addition, when CRT treatment is performed, it is possible to decide which party will incur the costs in a way that is acceptable for all the three parties concerned.

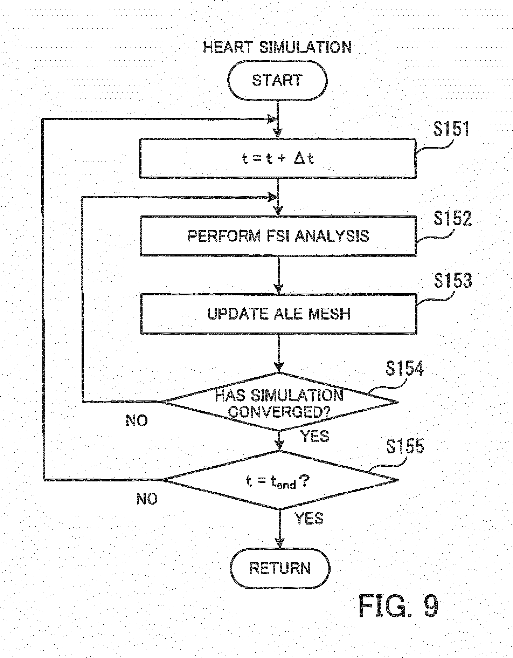

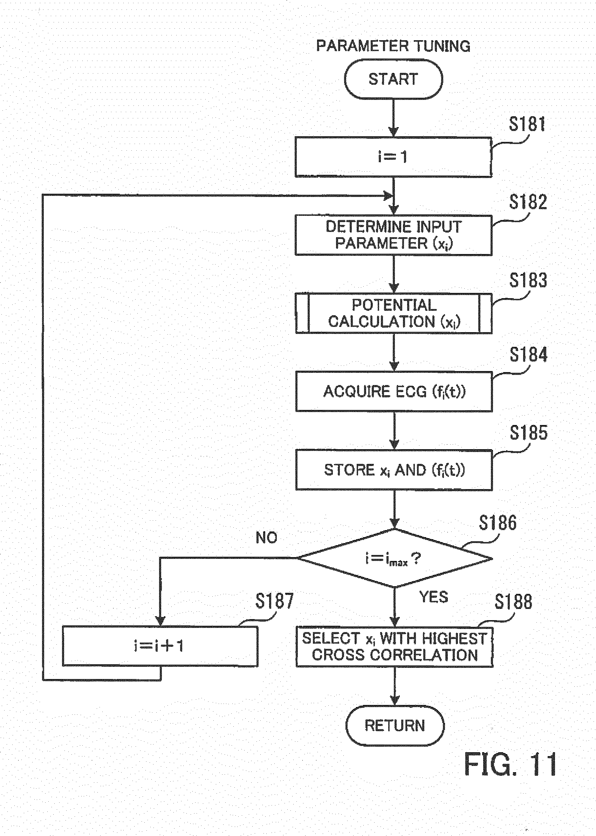

The parameter determining processing for heart simulation is described next in detail. The parameter determining processing is divided into a parameter determining process for dynamic system simulation ("dynamic system simulation parameter determining process") illustrated in FIGS. 8 and 9 and a parameter determining process for electrical system simulation ("electrical system simulation parameter determining process") illustrated in FIGS. 10 and 11.

The dynamic system simulation parameter determining process is described first. As illustrated in FIGS. 8 and 9, parameters for dynamic system simulation are determined by cooperation of the parameter determining unit 250 and the heart simulator 270. FIG. 8 is a flowchart illustrating an example of a processing procedure for determining dynamic system simulation parameters. The process of FIG. 8 is described according to the step numbers in the flowchart. Assume here that the heart model creating unit 240 has created morphology data representing the heart structure of the patient and simulation mesh data prior to initiating the dynamic system simulation parameter determining process.

[Step S141] The parameter determining unit 250 tunes parameters used in heart simulation. The parameters to be tuned include ones related to the stroke volume, blood pressure, infarction area, and the like. For example, the parameter determining unit 250 tunes the parameters in such a manner that heart simulation results obtained from a finite element mesh model approximate biomedical data (such as ECG data, blood pressure, echocardiography data, MRI/CT data, and catheterization data) actually obtained from the patient. The parameter tuning is made, for example, according to inputs provided by the doctor, in reference to previous simulation results.

[Step S142] The heart simulator 270 runs heart simulation using the created finite element mesh model and the tuned parameters. For example, the heart simulator 270 executes highly accurate cardiac fluid-structure interaction (FSI) simulation. Note that, in the FSI simulation, the heart simulator 270 may use mechanical heart valves or simulated heart valves in which rectifier circuits emulate the valves.

[Step S143] The parameter determining unit 250 performs a process of checking preoperative status. For example, the parameter determining unit 250 displays simulation results obtained from the finite element mesh model representing the patient's living heart of the moment and data on the patient's biomedical status of the moment, such as the stroke volume, in order to compare them quantitatively. If the simulation results differ from the current biomedical status of the patient, the parameters are tuned again based on, for example, doctor's instructions.

[Step S144] The heart simulator 270 displays behavior of the heart obtained as the simulation results. For example, based on the simulation results, the heart simulator 270 reproduces the behavior of the heart in animation.

The heart simulation of step S142 is described next in detail. FIG. 9 is a flowchart illustrating an example of a procedure for the heart simulation. The process of FIG. 9 is described according to the step numbers in the flowchart.

[Step S151] The heart simulator 270 updates the value of a time step. For example, the heart simulator 270 adds a predetermined time increment $\Delta t$ to the current time t. Note that the simulation start time is, for example, t = 0.

[Step S152] The heart simulator 270 performs FSI analysis established with a combination of an arbitrary Lagrangian-Eulerian (ALE) method and Lagrange's method of undetermined multipliers.

[Step S153] The heart simulator 270 updates an ALE mesh.

[Step S154] The heart simulator 270 determines whether calculation results have converged. If the result has converged, the procedure moves to step S155. If not, the procedure moves to step S152 and the calculation is executed again.

[Step S155] The heart simulator 270 determines whether the current simulation time t has reached an end time $t_{end}$. The end time $t_{end}$ is, for example, time spent on completing the simulation of one heartbeat. If the current time t has reached the end time $t_{end}$, the heart simulation ends. If the current time t has not reached the end time $t_{end}$, the procedure moves to step S151.

The electrical system simulation parameter determining process is described next in detail. FIG. 10 is a flowchart illustrating an example of a processing procedure for determining electrical system simulation parameters. The process of FIG. 10 is described according to the step numbers in the flowchart. Assume here that, prior to initiating the electrical system simulation parameter determining process, the heart model creating unit 240 has performed segmentation of the thorax (chest) and set electrical conductivities (for individual organs, fat, muscle, and bone), and has also performed segmentation of the heart and set an electrical conductivity of the heart. The electrical conductivity of an infarction area in the patient's heart is set with an assumption that the infarction area is not electrically excitable and does not conduct electric current.

[Step S161] The parameter determining unit 250 tunes the value of a parameter x for adjusting the QRS waves. The parameter x indicates, for example, an early excitation initiation site ("early excitation part"). For example, amongst a plurality of candidate values $x_i$ (i = 1, 2, 3, ..., and $i_{max}$) of the parameter x, the optimal value is selected. The parameter determining unit 250 adjusts the location of the early excitation part on the endocardial surface to thereby adjust the QRS waves. Details of the parameter tuning process are described later (see FIG. 11).

[Step S162] The parameter determining unit 250 determines whether the difference in the QRS interval (or a value corresponding to the difference) between a waveform f(t) on an ECG obtained from heart simulation using the tuned parameter and a measured waveform $f_{real}(t)$ on an ECG obtained from the patient is below a threshold. For example, the parameter determining unit 250 may divide the difference between f(t) and $f_{real}(t)$ by $f_{real}(t)$ and then determine whether the result of the division falls below the threshold. This is expressed by the following inequality: $|(f(t) - f_{real}(t))/f_{real}(t)| <$ threshold.

If the inequality above is true for each time t during the QRS complex, the procedure moves to step S163. If there is a time t for which the inequality is not true, the procedure moves to step S170.

[Step S163] The parameter determining unit 250 determines the parameter $x_i$ selected in step S161 as the value of the parameter x for adjusting the QRS waves.

[Step S164] The parameter determining unit 250 tunes the value of a parameter $y_i$ for adjusting a T wave. The parameter $y_i$ is a value, for example, indicating the distribution of cells with long action potential duration (APD) and cells with short APD in the ventricular myocardium. The APD is the amount of time in which the ventricular myocardium is contracting and corresponds to a part of the ECG waveform, starting from the QRS complex to the end of the T wave (QT interval). For example, the parameter determining unit 250 adjusts the T wave by adjusting the distribution of three cell models with different APDs (endocardial endothelial cells, epicaridal cells, and M (mid-myocardial) cells). The M cells are cardiac muscle cells, called cardiomyocytes, with the longest APD and distributed in the middle layer of the ventricular wall.

[Step S165] The parameter determining unit 250 determines whether the difference in the T wave (or a value corresponding to the difference) between a waveform f(t) on an ECG obtained from heart simulation using the tuned parameter and the measured waveform $f_{real}(t)$ on the ECG obtained from the patient always remains below a threshold. For example, the parameter determining unit 250 may divide the difference between f(t) and $f_{real}(t)$ by $f_{real}(t)$ and then determine whether the result of the division remains below the threshold. If the waveform difference in the T wave or the results of the division always remains below the threshold during the T wave, the procedure moves to step S166. If there is a period of time for which the waveform difference in the T wave or the result of the division exceeds or is equal to the threshold, the procedure moves to step S170.

[Step S166] The parameter determining unit 250 determines the parameter $y_i$ selected in step S164 as the value of the parameter for adjusting the T wave.

[Step S167] The parameter determining unit 250 tunes the value of a parameter $z_i$ for adjusting the amplitude of an ECG waveform. The parameter $z_i$ indicates, for example, thickness of subcutaneous fat on the body surface. For example, the parameter determining unit 250 adjusts the amplitude by setting the subcutaneous fat layer in the chest to be thin if the ECG amplitude is low on the whole and setting it to be thick if the ECG amplitude is generally high.

[Step S168] The parameter determining unit 250 determines whether the difference in the amplitude (or a value corresponding to the difference) between a waveform f(t) on an ECG obtained from heart simulation using the tuned parameter and the measured waveform $f_{real}(t)$ on the ECG obtained from the patient always remains below a threshold. For example, the parameter determining unit 250 may divide the difference between f(t) and $f_{real}(t)$ by $f_{real}(t)$ and then determine whether the result of the division remains below the threshold. If the waveform difference in the amplitude or the results of the division always remains below the threshold, the procedure moves to step S169. If there is a period of time for which the waveform difference in the amplitude or the result of the division exceeds or is equal to the threshold, the procedure moves to step S170.

[Step S169] The parameter determining unit 250 determines the parameter $z_i$ selected in step S167 as the value of the parameter for adjusting the amplitude. Subsequently, the electrical system simulation parameter determining process ends.

[Step S170] The parameter determining unit 250 determines that no proper parameter tuning has been made and ends the procedure. In this case, the CRT simulation managing unit 220 notifies the hospital information system 100 of the HSC 32 being not able to handle the patient concerned.

Details of the parameter tuning processes (steps S161, S164, and S167) are described next. FIG. 11 is a flowchart illustrating an example of a processing procedure for parameter tuning. The process of FIG. 11 is described according to the step numbers in the flowchart.

[Step S181] The parameter determining unit 250 sets a variable i to an initial value of 1.

[Step S182] The parameter determining unit 250 determines the $i^{th}$ parameter value $x_i$. Amongst prepared candidate parameter values, the $i^{th}$ value is determined as the parameter value $x_i$.

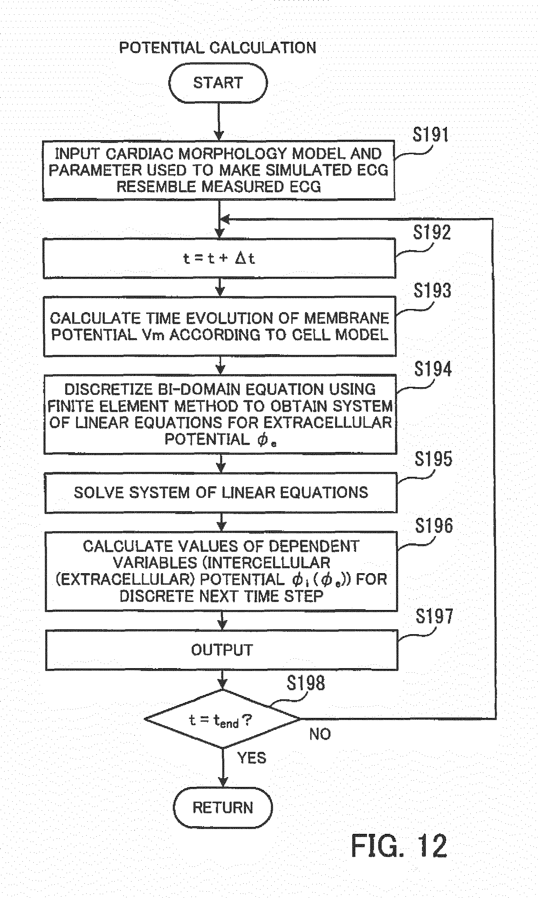

[Step S183] The parameter determining unit 250 requests the heart simulator 270 to calculate electrical potential for the parameter value $x_i$. Details of the electrical potential calculation are described later (see FIG. 12).

[Step S184] The parameter determining unit 250 acquires, from the heart simulator 270, the ECG $f_i(t)$ obtained as a result of simulation.

[Step S185] The parameter determining unit 250 stores, in memory, the ECG $f_i(t)$ in association with the parameter value $x_i$.

[Step S186] The parameter determining unit 250 determines whether the value of the variable i has reached the maximum value $i_{max}$. If it has not reached the maximum value $i_{max}$, the procedure moves to step S187. If it has reached the maximum value $i_{max}$, the procedure moves to step S188.

[Step S187] The parameter determining unit 250 adds 1 to the variable i, and then moves to step S182.

[Step S188] The parameter determining unit 250 selects, amongst all the parameter values $x_i$, one with the highest cross correlation as a result of the parameter tuning. In this regard, for example, a cross correlation $R_{NCC}$ ($-1 \leq R_{NCC} \leq 1$) is calculated by the following equation based on an ECG waveform A(k, j) obtained from heart simulation using the parameter $x_i$ and a measured ECG waveform B(k, j) obtained from the patient.

$$R_{NCC} = \frac{\sum_{j=1}^{12} \sum_{k=1}^{N} A(k,j) \times B(k,j)}{\sqrt{\sum_{j=1}^{12} \sum_{k=1}^{N} A(k,j)^2 \times \sum_{j=1}^{12} \sum_{k=1}^{N} B(k,j)^2}} \quad \cdots (1)$$

In the expression above, A(k, j) is the simulation result value (electrical potential) obtained at the $k^{th}$ (k = 1, 2, 3, ..., and N) simulation clock time in the $j^{th}$ ECG amongst 12-lead ECGs obtained from the heart simulation. B(k, j) is the value obtained at a time corresponding to the $k^{th}$ simulation clock time in the $j^{th}$ ECG amongst measured 12-lead ECGs obtained from the patient. A cross correlation of +1 indicates a perfect positive correlation; 0 indicates no correlation; and -1 indicates a perfect negative correlation. The parameter determining unit 250 selects $x_i$ with the highest cross correlation as the best parameter value. Subsequently, the parameter tuning process ends.

In the above-described manner, the best parameter value $x_i$ for adjusting the QRS waves is selected. If an ECG obtained from the heart simulation using the selected parameter $x_i$ satisfies the condition in step S162, the selected parameter value $x_i$ is determined as the value of the parameter for adjusting the QRS waves. Note that FIG. 11 illustrates an example of tuning the QRS-wave adjusting parameter; however, each of the T-wave and amplitude adjusting parameters may be tuned in the same manner.

Next the electrical potential calculating process is described in detail. FIG. 12 is a flowchart illustrating an example of a processing procedure for calculating electrical potentials. The process of FIG. 12 is described according to the step numbers in the flowchart.

[Step S191] The parameter determining unit 250 inputs to the heart simulator 270 the cardiac morphology model and the parameter used to make a simulated ECG waveform closely resemble the measured ECG waveform.

[Step S192] The heart simulator 270 updates the value of a time step. For example, the heart simulator 270 adds a predetermined time increment $\Delta t$ to the current time t. Note that the simulation start time is t = 0.

[Step S193] The heart simulator 270 calculates the time evolution of membrane potential Vm according to a cell model.

[Step S194] The heart simulator 270 discretizes an excitement propagation equation of a bi-domain model using a finite element method to obtain a system of linear equations for extracellular potential $\varphi_e$.

[Step S195] The heart simulator 270 solves the system of linear equations.

[Step S196] The heart simulator 270 calculates values of dependent variables, intercellular potential $\varphi_i$ and the extracellular potential $\varphi_e$, for a discrete next time step.

[Step S197] The heart simulator 270 outputs the calculated dependent variable values. The output values are stored in memory.

[Step S198] The heart simulator 270 determines whether the current simulation time t has reached an end time $t_{end}$. The end time $t_{end}$ is, for example, time spent on completing the simulation of one heartbeat. If the current time t has reached the end time $t_{end}$, the electrical potential calculating process ends. If the current time t has not reached the end time $t_{end}$, the procedure moves to step S192.

By the processes of FIGS. 8 to 12, the parameters for accurately reproducing the patient's ECG in heart simulation are determined. The heart simulation executed using the determined parameters is considered to accurately replicate real-life behavior of the patient's heart before CRT implantation. Therefore, it is possible to accurately predict heart behavior of the patient following the CRT implantation by running heart simulation that models electrical signals sent from a CRT device to the patient's heart, using the determined parameters.

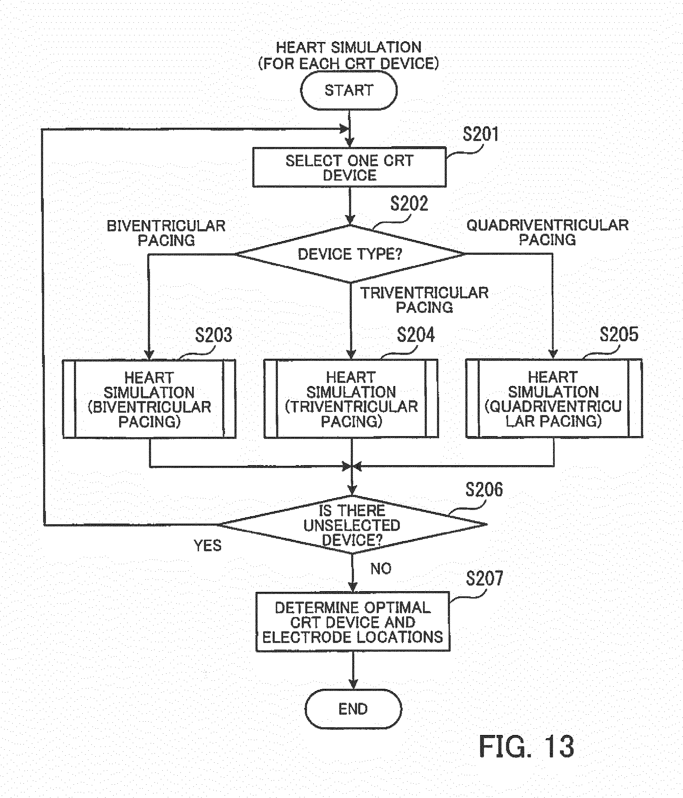

Heart simulation run for each CRT device in order to determine the optimal CRT device and electrode locations is described next in detail. FIG. 13 is a flowchart illustrating an example of a processing procedure of the heart simulation for each CRT device. The process of FIG. 13 is described according to the step numbers in the flowchart.

[Step S201] The heart simulator 270 selects one CRT device subject to the simulation. For example, the heart simulator 270 selects one CRT device from the CRT device list of the CRT device list storing unit 210.

[Step S202] The heart simulator 270 determines the type of the selected CRT device. For example, if an electrode disposition pattern input thereto includes two electrodes placed in the ventricles, the heart simulator 270 determines the device type as "biventricular pacing". Similarly, in the case of three and four electrodes placed in the ventricles, the device type is determined to be "triventricular pacing" and "quadriventricular pacing", respectively. When the device type is biventricular pacing, the procedure moves to step S203. When the device type is triventricular pacing, the procedure moves to step S204. When the device type is quadriventricular pacing, the procedure moves to step S205.

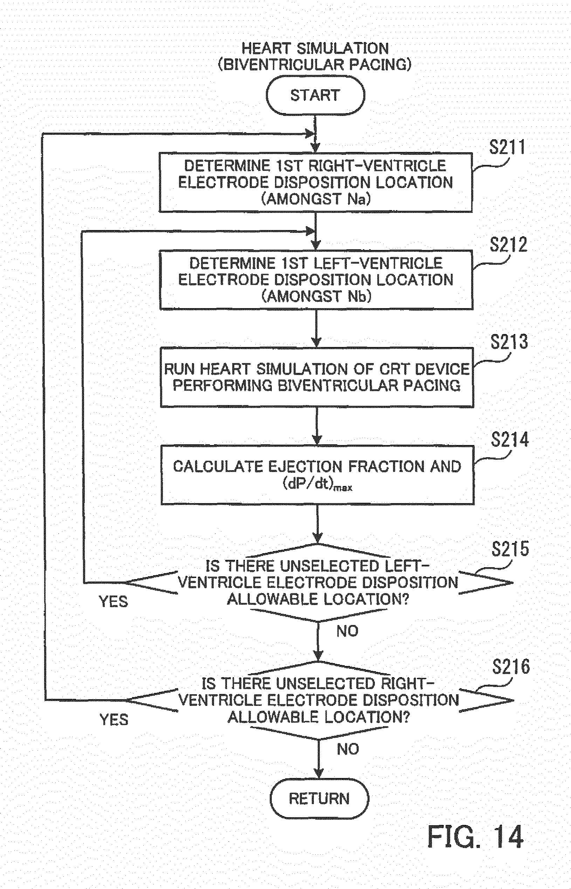

[Step S203] The heart simulator 270 runs heart simulation designed for a biventricular pacing CRT device. Details of this process are described later (see FIG. 14). Subsequently, the procedure moves to step S206.

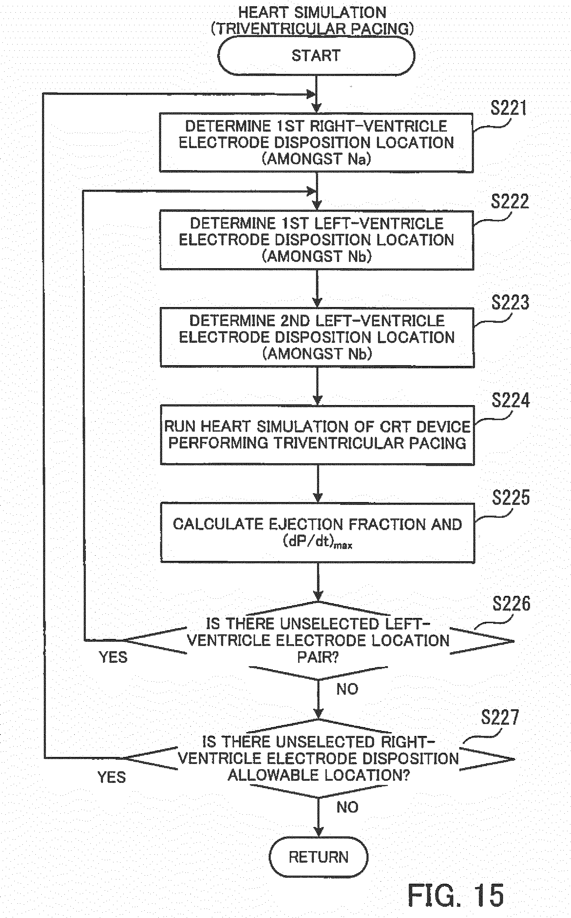

[Step S204] The heart simulator 270 runs heart simulation designed for a triventricular pacing CRT device. Details of this process are described later (see FIG. 15). Subsequently, the procedure moves to step S206.

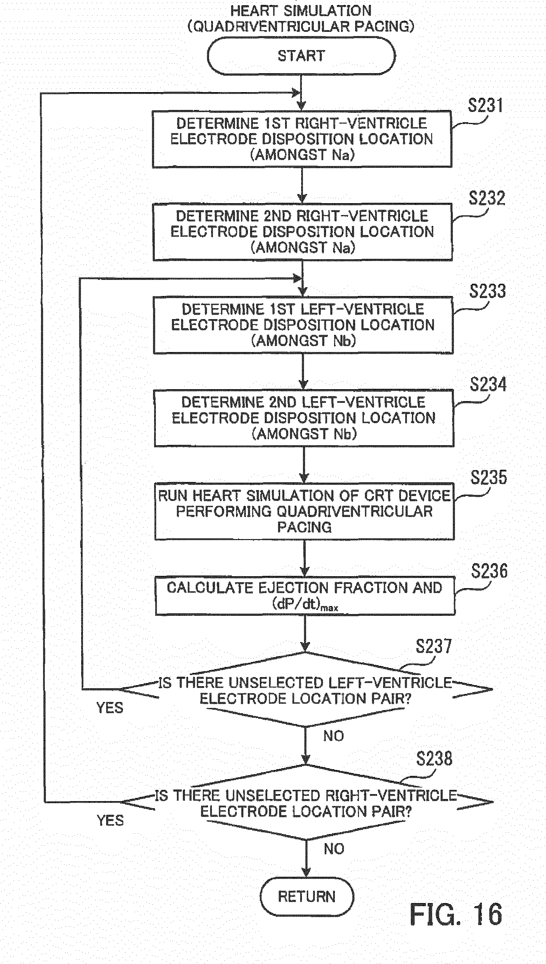

[Step S205] The heart simulator 270 runs heart simulation designed for a quadriventricular pacing CRT device. Details of this process are described later (see FIG. 16). Subsequently, the procedure moves to step S206.

[Step S206] The heart simulator 270 determines whether there is an unselected CRT device. If there is an unselected CRT device, the procedure moves to step S201. If not, the procedure moves to step S207.

[Step S207] Based on results obtained from the simulation for individual electrode disposition patterns of each CRT device, the CRT simulation managing unit 220 determines a CRT device and its electrode locations yielding the highest improvement rate. For example, the CRT simulation managing unit 220 selects, as the first candidate pattern, an electrode disposition pattern with the highest rate of improvement in the ejection fraction and, as the second candidate pattern, an electrode disposition pattern with the highest rate of improvement in $(dP/dt)_{max}$. Then, between the first candidate pattern with the rate of improvement in the ejection fraction and the second candidate pattern with the rate of improvement in $(dP/dt)_{max}$, the CRT simulation managing unit 220 selects one with a higher improvement rate, and determines a CRT device and its electrode locations associated with the selected candidate pattern as the optimal CRT device and electrode locations.

Assume for example that, in steps S203 to S205, the heart simulation has been executed for N different electrode disposition patterns, and the electrode disposition patterns are numbered serially, starting from 1. The ejection fraction and $(dP/dt)_{max}$ of the $i^{th}$ electrode disposition pattern (i = 1, ..., and N) are denoted by $EF_i$ and $(dP_i/dt)_{max}$, respectively. A maximum improvement rate $IR_{max}$ is expressed as:

$$IR_{Max} = \{Max((EF_i/EF_e):i = 0, ..., N),$$

$$Max((dP_i/dt)_{max}/(dP_e/dt)_{max}:i = 0, ..., N)\}$$

where $EF_e$ is the ejection fraction before treatment, and $(dP_e/dt)_{max}$ is $(dP/dt)_{max}$ before treatment. A CRT device and electrode locations associated with an electrode disposition pattern yielding the maximum improvement rate are the optimal CRT device and electrode locations.

In the above-described procedure, the optimal CRT device and electrode locations are determined. Details of the individual processes (steps S203 to S205) of FIG. 13 are described next. Assume, in the following description, that there are Na locations (Na is an integer greater than or equal to 1) in the right ventricle, allowed for electrode disposition (hereinafter referred to as "right-ventricle electrode disposition allowable locations") and Nb locations (Nb is an integer greater than or equal to 1) in the left ventricle, allowed for electrode disposition ("left-ventricle electrode disposition allowable locations").

First the heart simulation of a CRT device performing biventricular pacing is described. FIG. 14 illustrates an example of a procedure of the heart simulation designed for a biventricular pacing CRT device. The process of FIG. 14 is described according to the step numbers in the flowchart.

[Step S211] The heart simulator 270 selects, amongst the Na right-ventricle electrode disposition allowable locations, one unselected location (i.e., a location which has not yet been selected for electrode disposition), and determines it as the first electrode disposition location in the right ventricle.

At this point, the heart simulator 270 resets the state of all the left-ventricle electrode disposition allowable locations to "unselected" and then moves to step S212.

[Step S212] The heart simulator 270 selects, amongst the Nb left-ventricle electrode disposition allowable locations, one unselected location, and determines it as the first electrode disposition location in the left ventricle.

[Step S213] Based on the determined electrode disposition locations, the heart simulator 270 runs heart simulation of the CRT device performing biventricular pacing.

[Step S214] The heart simulator 270 calculates the ejection fraction and $(dP/dt)_{max}$ based on results of the simulation, and then stores, in memory, the ejection fraction and $(dP/dt)_{max}$ in association with an electrode disposition pattern composed of the electrode disposition locations individually determined in steps S211 and S212.

[Step S215] The heart simulator 270 determines whether there is an unselected left-ventricle electrode disposition allowable location. If there is an unselected left-ventricle electrode disposition allowable location, the procedure moves to step S212. If not, the procedure moves to step S216.

[Step S216] The heart simulator 270 determines whether there is an unselected right-ventricle electrode disposition allowable location. If there is an unselected right-ventricle electrode disposition allowable location, the procedure moves to step S211. If not, the heart simulation ends.

Next the heart simulation of a CRT device performing triventricular pacing is described. FIG. 15 illustrates an example of a procedure of the heart simulation designed for a triventricular pacing CRT device. The process of FIG. 15 is described according to the step numbers in the flowchart.

[Step S221] The heart simulator 270 selects, amongst the Na right-ventricle electrode disposition allowable locations, one unselected location, and determines it as the first electrode disposition location in the right ventricle.

At this point, the heart simulator 270 resets the state of all combinations of two locations ("electrode location pairs") selected amongst the Nb left-ventricle electrode disposition allowable locations to "unselected" and then moves to step S222.

[Step S222] The heart simulator 270 selects one unselected electrode location pair in the left ventricle, and determines one of the electrode disposition allowable locations included in the selected electrode location pair as the first electrode disposition location in the left ventricle.

[Step S223] The heart simulator 270 determines the other electrode disposition allowable location of the electrode location pair selected in step S222 as the second electrode disposition location in the left ventricle.

[Step S224] Based on the determined electrode disposition locations, the heart simulator 270 runs heart simulation of the CRT device performing triventricular pacing.

[Step S225] The heart simulator 270 calculates the ejection fraction and $(dP/dt)_{max}$ based on results of the simulation, and then stores, in memory, the ejection fraction and $(dP/dt)_{max}$ in association with an electrode disposition pattern composed of the electrode disposition locations individually determined in steps S221 to S223.

[Step S226] The heart simulator 270 determines whether there is an unselected left-ventricle electrode location pair. If there is an unselected left-ventricle electrode location pair, the procedure moves to step S222. If not, the procedure moves to step S227.

[Step S227] The heart simulator 270 determines whether there is an unselected right-ventricle electrode disposition allowable location. If there is an unselected right-ventricle electrode disposition allowable location, the procedure moves to step S221. If not, the heart simulation ends.

Next the heart simulation of a CRT device performing quadriventricular pacing is described. FIG. 16 illustrates an example of a procedure of the heart simulation designed for a quadriventricular pacing CRT device. The process of FIG. 16 is described according to the step numbers in the flowchart.

[Step S231] The heart simulator 270 selects, out of all combinations of two locations ("electrode location pairs") selected amongst the Na right-ventricle electrode disposition allowable locations, one unselected electrode location pair. Then, the heart simulator 270 determines one of the electrode disposition allowable locations included in the selected electrode location pair as the first electrode disposition location in the right ventricle.

[Step S232] The heart simulator 270 determines the other electrode disposition allowable location of the electrode location pair selected in step S231 as the second electrode disposition location in the right ventricle.

At this point, the heart simulator 270 resets the state of all the left-ventricle electrode location pairs to "unselected" and then moves to step S233.

[Step S233] The heart simulator 270 selects one unselected electrode location pair in the left ventricle, and determines one of the electrode disposition allowable locations included in the selected electrode location pair as the first electrode disposition location in the left ventricle.

[Step S234] The heart simulator 270 determines the other electrode disposition allowable location of the electrode location pair selected in step S233 as the second electrode disposition location in the left ventricle.

[Step S235] Based on the determined electrode disposition locations, the heart simulator 270 runs heart simulation of the CRT device performing quadriventricular pacing.

[Step S236] The heart simulator 270 calculates the ejection fraction and $(dP/dt)_{max}$ based on results of the simulation, and then stores, in memory, the ejection fraction and $(dP/dt)_{max}$ in association with an electrode disposition pattern composed of the electrode disposition locations individually determined in steps S231 to S234.

[Step S237] The heart simulator 270 determines whether there is an unselected left-ventricle electrode location pair. If there is an unselected left-ventricle electrode location pair, the procedure moves to step S233. If not, the procedure moves to step S238.

[Step S238] The heart simulator 270 determines whether there is an unselected left-ventricle electrode location pair. If there is an unselected left-ventricle electrode location pair, the procedure moves to step S231. If not, the heart simulation ends.

By the processes of FIGS. 13 to 16, the heart simulation is run for various electrode disposition patterns with respect to each CRT device. Then, a CRT device and electrode locations associated with an electrode disposition pattern yielding the maximum improvement rate are determined as the optimal CRT device and electrode locations.

As for indexes used to compare the improvement rates, at least one of the following may be used: left ventricle end-systolic volume (LVESV); stroke volume (SV); improvement in mitral regurgitation (MR); pulse pressure; improvement in interventricular (inter-V) asynchrony; and ejection time (ET). LVESV is the volume of blood in the left ventricle at the time of maximal cardiac contraction.

Mitral regurgitation (MR) is leakage of blood backwards (regurgitation) through the mitral valve each time the left ventricle contracts. When mitral regurgitation is present, some blood leaks backward into the left atrium as the left ventricle pumps blood into the aorta, increasing blood volume and internal pressure in the left atrium. The increased blood pressure in the left atrium elevates blood pressure in the pulmonary veins leading from the lungs to the heart, and causes the left atrium to enlarge to accommodate the extra blood leaking back from the ventricle. An extremely enlarged atrium often beats rapidly in an irregular pattern (a disorder called atrial fibrillation), which reduces the cardiac pumping efficiency because the fibrillating atrium is just quivering rather than pumping. Consequently, blood does not flow through the atrium normally, and blood clots may form inside the chamber. If a clot breaks loose and becomes an embolus, it is pumped out of the heart and may block an artery, possibly causing a stroke or damage to another organ. Severe regurgitation may result in heart failure, in which increased pressure in the atrium causes fluid accumulation (congestion) in the lungs, or in which reduced forward flow of blood from the ventricle to the body deprives organs from the proper amount of blood. The left ventricle may gradually dilate and weaken, further worsening heart failure. Therefore, if a patient with mitral regurgitation benefits symptomatically from treatment by CRT implantation, there is good reason for performing the CRT implantation. Thus, an improvement in mitral regurgitation is considered as one of critical indexes to determine the effectiveness of the CRT treatment.

The pulse pressure is the difference between the systolic and diastolic blood pressure. The pulse pressure increases with increased stroke volume and decreases with an increase in the volume of the arterial system. In addition, the pulse pressure also increases due to arteriosclerosis. The pulse pressure is used in diagnosis of hypertension and the like together with systolic and diastolic blood pressure and serves as an effective index to determine the cardiac condition. The inter-V asynchrony is information indicating asynchronous beating of the left and right ventricles. The ejection time is the time taken to eject the blood from the ventricle.

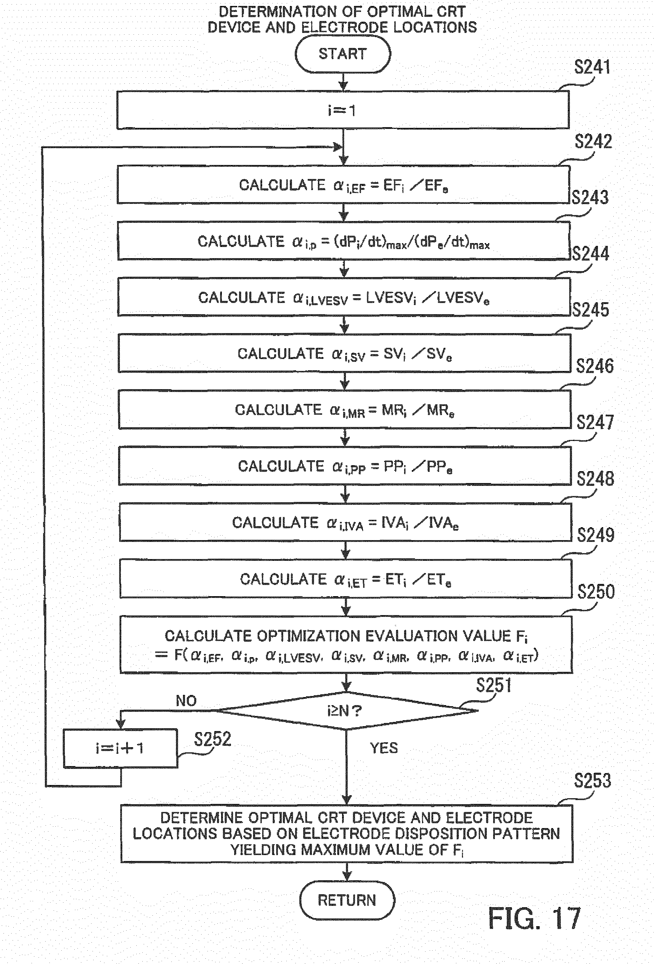

A combination of all the various indexes may be used to determine the optimal CRT device and electrode locations. FIG. 17 is a flowchart illustrating an example of a processing procedure for determining the optimal CRT device and electrode locations based on various indexes. Assume in this example that heart simulation has been executed for N different electrode disposition patterns. The ejection fraction and $(dP/dt)_{max}$ of the $i^{th}$ electrode disposition pattern ($i = 1, ...,$ and N) are denoted by and $(dP_i/dt)_{max}$, respectively. Further, the improvement rates of the individual indexes are denoted as follows:

- improvement rate of the ejection fraction of the $i^{th}$ electrode disposition pattern: $\alpha_{i,EF} = EF_i/EF_e$ (where $EF_i$ is the ejection fraction of the $i^{th}$ electrode disposition pattern, and $EF_e$ is the ejection fraction before treatment);
- improvement rate of $(dP/dt)_{max}$ (pressure change rate) of the $i^{th}$ electrode disposition pattern: $\alpha_{i,p} = (dP_i/dt)_{max}/(dP_e/dt)_{max}$
  (where $(dP_i/dt)_{max}$ is $(dP/dt)_{max}$ of the $i^{th}$ electrode disposition pattern, and $(dP_e/dt)_{max}$ is $(dP/dt)_{max}$ before treatment);
- improvement rate of LVESV of the $i^{th}$ electrode disposition pattern: $\alpha_{i,LVESV} = LVESV_i/LVESV_e$
  (where $LVESV_i$ is LVESV of the $i^{th}$ electrode disposition pattern, and $LVESV_e$ is LVESV before treatment);
- improvement rate of the stroke volume of the $i^{th}$ electrode disposition pattern: $\alpha_{i,SV} = SV_i/SV_e$
  (where $SV_i$ is the stroke volume of the $i^{th}$ electrode disposition pattern, and $SV_e$ is the stroke volume before treatment);
- improvement rate of the mitral regurgitation of the $i^{th}$ electrode disposition pattern: $\alpha_{i,MR} = MR_i/MR_e$ (where $MR_i$ is the mitral regurgitation of the $i^{th}$ electrode disposition pattern, and $MR_e$ is the mitral regurgitation before treatment);
- improvement rate of the pulse pressure of the $i^{th}$ electrode disposition pattern: $\alpha_{i,PP} = PP_i/PP_e$
  (where $PP_i$ is the pulse pressure of the $i^{th}$ electrode disposition pattern, and $PP_e$ is the pulse pressure before treatment);
- improvement rate of the inter-V asynchrony of the $i^{th}$ electrode disposition pattern: $\alpha_{i,IVA} = IVA_i/IVA_e$ (where $IVA_i$ is the inter-V asynchrony of the $i^{th}$ electrode disposition pattern, and $IVA_e$ is the inter-V asynchrony before treatment); and
- improvement rate of the ejection time of the $i^{th}$ electrode disposition pattern: $\alpha_{i,ET} = ET_i/ET_e$
  (where $ET_i$ is the ejection time of the $i^{th}$ electrode disposition pattern, and $ET_e$ is the ejection time before treatment).

The process of FIG. 17 is described next according to the step numbers in the flowchart.

[Step S241] The CRT simulation managing unit 220 sets the variable i for designating an electrode disposition pattern to an initial value of 1.

[Step S242] The CRT simulation managing unit 220 calculates the improvement rate of the ejection fraction of the $i^{th}$ electrode disposition pattern, "$\alpha_{i,EF} = EF_i/EF_e$".

[Step S243] The CRT simulation managing unit 220 calculates the improvement rate of $(dP/dt)_{max}$ (pressure change rate) of the $i^{th}$ electrode disposition pattern, "$\alpha_{i,p} = (dP_i/dt)_{max}/(dP_e/dt)_{max}$".

[Step S244] The CRT simulation managing unit 220 calculates the improvement rate of the LVESV of the $i^{th}$ electrode disposition pattern, "$\alpha_{i,LVESV} = LVESV_i/LVESV_e$".

[Step S245] The CRT simulation managing unit 220 calculates the improvement rate of the stroke volume of the $i^{th}$ electrode disposition pattern, "$\alpha_{i,SV} = SV_i/SV_e$".

[Step S246] The CRT simulation managing unit 220 calculates the improvement rate of the mitral regurgitation of the $i^{th}$ electrode disposition pattern, "$\alpha_{i,MR} = MR_i/MR_e$".

[Step S247] The CRT simulation managing unit 220 calculates the improvement rate of the pulse pressure of the $i^{th}$ electrode disposition pattern, "$\alpha_{i,PP} = PP_i/PP_e$".

[Step S248] The CRT simulation managing unit 220 calculates the improvement rate of the inter-V asynchrony of the $i^{th}$ electrode disposition pattern, "$\alpha_{i,IVA} = IVA_i/IVA_e$".

Step S249] The CRT simulation managing unit 220 calculates the improvement rate of the ejection time of the $i^{th}$ electrode disposition pattern, "$\alpha_{i,ET} = ET_i/ET_e$".

[Step S250] The CRT simulation managing unit 220 calculates an optimization evaluation value $F_i$ of the $i^{th}$ electrode disposition pattern using an optimization evaluation function $F(\alpha_{i,EF}, \alpha_{i,p}, \alpha_{i,LVESV}, \alpha_{i,SV}, \alpha_{i,MR}, \alpha_{i,PP}, \alpha_{i,IVA},$

$\alpha_{i,ET}$) of the improvement rates of the individual indexes of the $i^{th}$ electrode disposition pattern. The optimization evaluation function is, for example, the following weighted average function:

$$F_i = \beta_{EF}\alpha_{i,EF} + \beta_P\alpha_{i,P} + \beta_{LVESV}\alpha_{i,LVESV} + \beta_{SV}\alpha_{i,SV} + \beta_{MR}\alpha_{i,MR} + \beta_{PP}\alpha_{i,PP} + \beta_{IVA}\alpha_{i,IVA} + \beta_{ET}\alpha_{i,ET}$$

where $\beta_{EF}$, $\beta_P$, $\beta_{LVESV}$, $\beta_{SV}$, $\beta_{MR}$, $\beta_{PP}$, $\beta_{IVA}$, and $\beta_{ET}$ are weight values for the individual indexes of the $i^{th}$ electrode disposition pattern. Each of the weight values is a number greater than 0 but smaller than 1. These weight values are set in advance from a medical perspective.

[Step S251] The CRT simulation managing unit 220 determines whether the variable i is N or more. If the variable i is N or more, the procedure moves to step S253. If the variable i is below N, the procedure moves to step S252.

[Step S252] The CRT simulation managing unit 220 adds 1 to the value of the variable i and then moves to step S242.

[Step S253] The CRT simulation managing unit 220 determines a CRT device and electrode locations associated with an electrode disposition pattern yielding the maximum value of $F_i$ (i = 1, ..., and N) as the optimal CRT device and electrode locations.

In the above-described manner, the optimization evaluation value is calculated for each electrode disposition pattern using the predefined optimization evaluation function. Then, a CRT device and electrode locations associated with an electrode disposition pattern yielding the maximum optimization evaluation value are determined as the optimal CRT device and electrode locations.

According to the second embodiment, the CRT simulation enables accurate determination of the optimal CRT device for the patient and the optimal electrode locations for implanting the CRT device. Then, if the improvement rate obtained with the optimal CRT device and electrode locations is greater than or equal to a specified value, the CRT implantation is determined to be effective to the patient. The determination result on the effectiveness of the CRT implantation is shared among the doctor, the patient, and the healthcare insurance company 33. Therefore, based on the accurate information, appropriate decisions are made about whether to provide CRT implantation and which party to incur the medical treatment costs. This results in preventing unnecessary CRT implantation and reducing medical expenses.

(c) Third Embodiment

Next described is a third embodiment. The third embodiment is directed to giving primary importance to doctor's determination in deciding whether to make insurance reimbursement. The third embodiment differs from the second embodiment in the following point. According to the second embodiment, the healthcare insurance company 33 determines whether to reimburse the medical treatment costs based on the simulation results. However, adding a doctor's opinion to the simulation results would give a more accurate judgment on the effectiveness of the CRT treatment. In view of this, according to the third embodiment, only a doctor's determination result is sent to the healthcare insurance company 33.

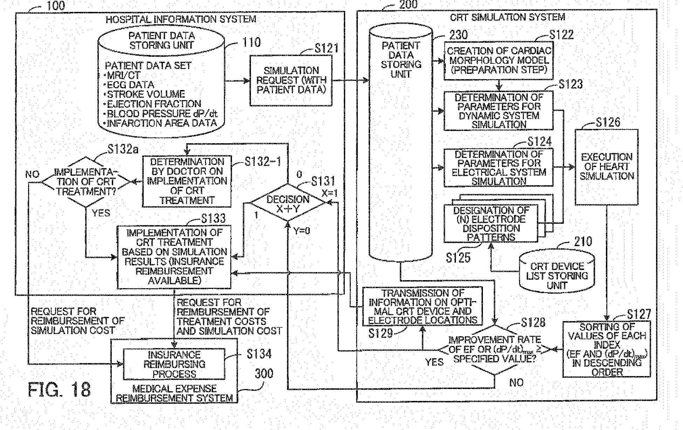

FIG. 18 illustrates an example of a procedure of CRT treatment and medical expense reimbursement according to the third embodiment. According to the third embodiment, if the improvement rate predicted by the CRT simulation system 200 is determined to fall below the specified value (step S128), the determination result is sent to the hospital information system 100. In the hospital information system 100, the CRT simulation request daemon 120 plugs in 0 for the variable Y based on the determination result of the improvement rate being below the specified value. Subsequently, the effectiveness of the CRT treatment is determined based on the simulation results (step S131), as in the case of the second embodiment. When the CRT treatment is expected to provide no benefit, whether to perform the CRT treatment is determined by the doctor (step S132-1). At this point, the cost managing unit 130 bifurcates the process according to the determination of the doctor on the CRT treatment (step S132a). If the doctor determines to perform the CRT treatment, the doctor then carries out the CRT treatment based on the simulation results (for example, using the optimal CRT device and electrode locations) (step S133). Subsequently, the cost managing unit 130 sends a request for reimbursement of the medical treatment costs and simulation cost to the medical expense reimbursement system 300. On the other hand, if the doctor determines not to perform the CRT treatment, the cost managing unit 130 sends a request for reimbursement of the simulation cost to the medical expense reimbursement system 300. According to the request from the hospital information system 100, the reimbursement processing unit 310 of the medical expense reimbursement system 300 performs the process of reimbursing the medical treatment costs and/or the simulation cost (step S134).

Thus, according to the third embodiment, the simulation results are sent to the hospital information system 100 and a final decision on whether to perform the CRT treatment is then made by the doctor. The healthcare insurance company 33 trusts the feedback from the doctor and makes the insurance reimbursement.

(d) Fourth Embodiment

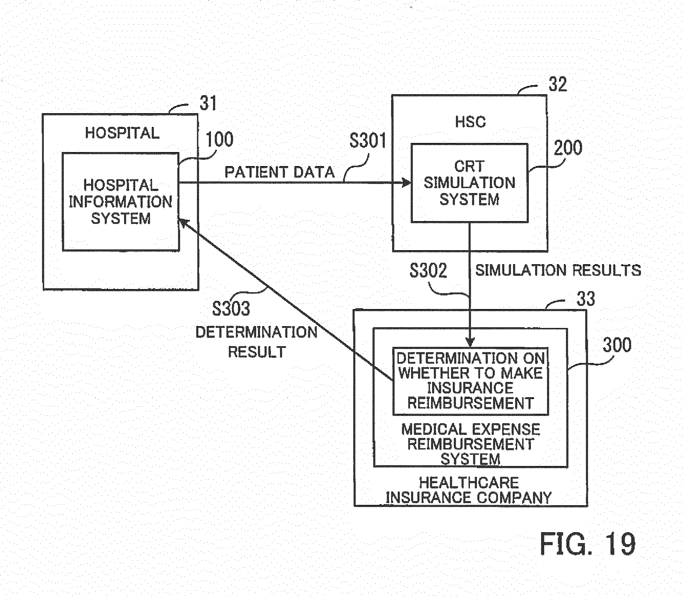

Next described is a fourth embodiment. According to the fourth embodiment, the healthcare insurance company 33 determines whether the implementation of the CRT treatment provides benefits based on the CRT simulation results. FIG. 19 illustrates an example of a processing procedure according to the fourth embodiment. A simulation request together with patient data, such as medical images and ECG data, is sent from the hospital information system 100 of the hospital 31 to the CRT simulation system 200 of the HSC 32 (step S301). Then, the heart simulation results obtained by the CRT simulation system 200 are sent not to the hospital information system 100 but to the medical expense reimbursement system 300 of the healthcare insurance company 33 (step S302). Subsequently, whether to make insurance reimbursement is determined by the medical expense reimbursement system 300 and the determination result is sent to the hospital information system 100 (step S303).

The fourth embodiment differs from the second embodiment in the following point. According to the second embodiment, the CRT simulation request daemon 120 of the hospital information system 100 decides whether the implementation of the CRT treatment provides benefits based on the simulation results; however, this decision is made by the medical expense reimbursement system 300 according to the fourth embodiment.

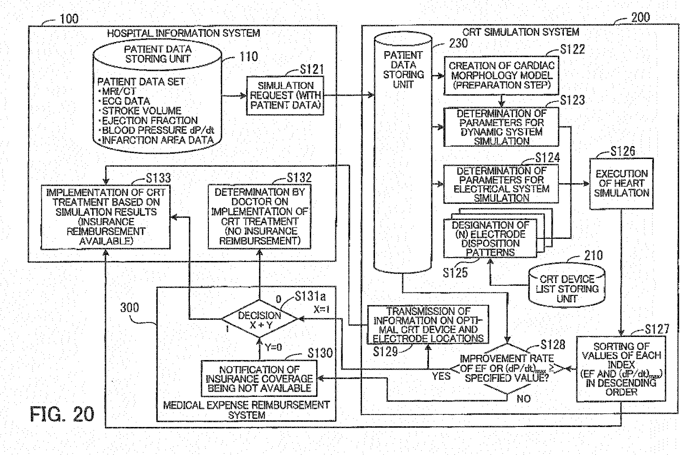

FIG. 20 illustrates an example of a procedure of CRT treatment and medical expense reimbursement according to the fourth embodiment. According to the fourth embodiment, if the improvement rate predicted by the CRT simulation system 200 is determined to be greater than or equal to the specified value (step S128), the determination result is sent to the medical expense reimbursement system 300. Upon receiving the determination result from the CRT simulation system 200, the reimbursement processing unit 310 of the medical expense reimbursement system 300 plugs in 1 for the variable X. On the other hand, if the reimbursement processing unit 310 receives a determination result indicating that the improvement rate falls below the specified value from the CRT simulation system 200, the reimbursement processing unit 310 outputs notice of the insurance coverage for the CRT treatment being unavailable (step S130) and plugs in 0 for the variable Y. Then, the reimbursement processing unit 310 calculates the value obtained by X + Y to thereby determine whether the implementation of the CRT treatment provides benefits (step S131a). If X + Y = 1, the reimbursement processing unit 310 notifies the hospital information system 100 that insurance reimbursement for the CRT treatment will be made. If X + Y = 0, the reimbursement processing unit 310 notifies the hospital information system 100 that no insurance reimbursement will be available for the CRT treatment.

Thus, according to the fourth embodiment, the healthcare insurance company 33 receives the simulation results and the determination result directly from the CRT simulation system 200 and then presents these results to the hospital 31. This allows the healthcare insurance company 33 to determine whether to make insurance reimbursement for the CRT treatment before the CRT treatment is carried out, which facilitates a quick reimbursement procedure.

(e) Fifth Embodiment

Next described is a fifth embodiment. According to the fifth embodiment, the healthcare insurance company 33 makes a primary decision on whether to perform the CRT treatment; however, a final decision thereon is made by the doctor, and the healthcare insurance company 33 respects the doctor's decision and reimburses the medical treatment costs.

The fifth embodiment differs from the third embodiment in the following point. According to the third embodiment, the CRT simulation request daemon 120 of the hospital information system 100 decides whether the implementation of the CRT treatment provides benefits based on the simulation results; however, this decision is made by the medical expense reimbursement system 300 according to the fifth embodiment.

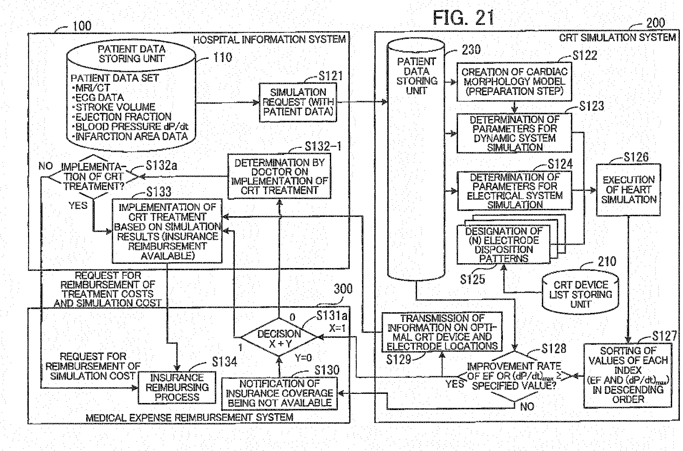

FIG. 21 illustrates an example of a procedure of CRT treatment and medical expense reimbursement according to the fifth embodiment. According to the fifth embodiment, if the improvement rate predicted by the CRT simulation system 200 is determined to be greater than or equal to the specified value (step S128), the determination result is sent to the medical expense reimbursement system 300. Upon receiving the determination result from the CRT simulation system 200, the reimbursement processing unit 310 of the medical expense reimbursement system 300 plugs in 1 for the variable X. On the other hand, if the reimbursement processing unit 310 receives a determination result indicating that the improvement rate falls below the specified value from the CRT simulation system 200, the reimbursement processing unit 310 outputs notice of the insurance coverage for the CRT treatment being unavailable (step S130) and plugs in 0 for the variable Y. Then, the reimbursement processing unit 310 calculates the value obtained by X + Y to thereby determine whether the implementation of the CRT treatment provides benefits (step S131a). If X + Y = 1, the reimbursement processing unit 310 notifies the hospital information system 100 that insurance reimbursement for the CRT treatment will be made. If X + Y = 0, the reimbursement processing unit 310 notifies the hospital information system 100 that no insurance reimbursement will be available for the CRT treatment. According to the fifth embodiment, however, even if the reimbursement processing unit 310 has primarily determined not to reimburse the CRT treatment, the reimbursement processing unit 310 performs the insurance reimbursement process if the doctor decides to perform the CRT treatment (step S134).

Thus, according to the fifth embodiment, even if the improvement rate obtained from the CRT simulation falls below the predetermined value, if the doctor at the hospital 31 determines it appropriate to implement the CRT treatment, the healthcare insurance company 33 trusts the determination of the doctor. When receiving a request for reimbursement of the medical treatment costs and simulation cost from the hospital information system 100, the medical expense reimbursement system 300 makes insurance reimbursement for these costs.

(f) Sixth Embodiment

Next described is a sixth embodiment. According to the sixth embodiment, the healthcare insurance company 33 conducts analysis of the effectiveness of the CRT treatment, such as calculation of the improvement rate. The sixth embodiment differs from the second embodiment in the following point. According to the second embodiment, the CRT simulation system 200 determines the optimal CRT device and electrode locations and whether the improvement rate achieved using the optimal CRT device and electrode locations is greater than or equal to the specified value. According to the sixth embodiment, however, these processes are performed by the medical expense reimbursement system 300.

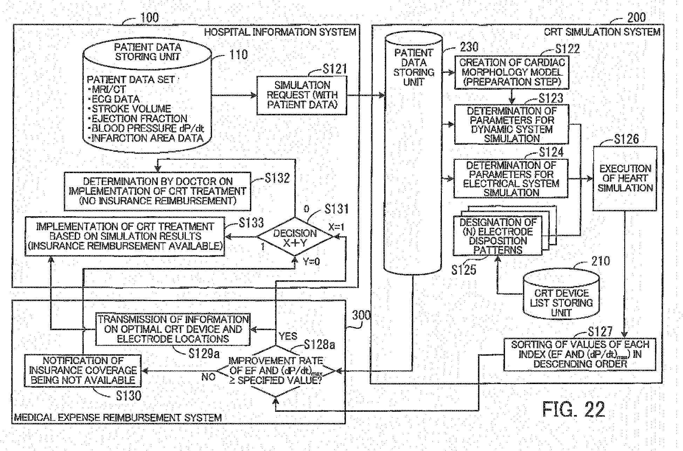

FIG. 22 illustrates an example of a procedure of CRT treatment and medical expense reimbursement according to the sixth embodiment. According to the sixth embodiment, the CRT simulation managing unit 220 of the CRT simulation system 200 sends the results of the heart simulation (such as the ejection fraction and $(dP/dt)_{max}$) to the medical expense reimbursement system 300. In the medical expense reimbursement system 300, the reimbursement processing unit 310 refers to the patient data storing unit 230 of the CRT simulation system 200 and determines whether the improvement rate of the optimal CRT device and electrode locations is greater than or equal to the specified value (step S128a). Then, if the improvement rate is greater than or equal to the specified value, the reimbursement processing unit 310 sends information on the optimal CRT device and electrode locations to the hospital information system 100 (step S129a).

Thus, according to the sixth embodiment, the determination on whether the improvement rate is greater than or equal to the specified value is made not by the CRT simulation system 200 but by the medical expense reimbursement system 300. This allows the healthcare insurance company 33 to analyze the improvement rate providing an indication of insurance reimbursement and statistically understand how high or low the specified value for the improvement rate needs to be set to achieve the insurance reimbursement. As a result, it becomes easier for the healthcare insurance company 33 to determine proper insurance premiums and set an appropriate specified value for the improvement rate.

(g) Seventh Embodiment

Next described is a seventh embodiment. According to the seventh embodiment, although the healthcare insurance company 33 conducts the analysis of the effectiveness of the CRT treatment, such as calculation of the improvement rate, it allows the doctor to make a final decision on whether to perform the CRT treatment, and trusts the feedback from the doctor and makes insurance reimbursement. The seventh embodiment differs from the sixth embodiment in the following point. The seventh embodiment includes a function of reimbursing the medical treatment costs according to the doctor's determination on whether to perform the CRT treatment.

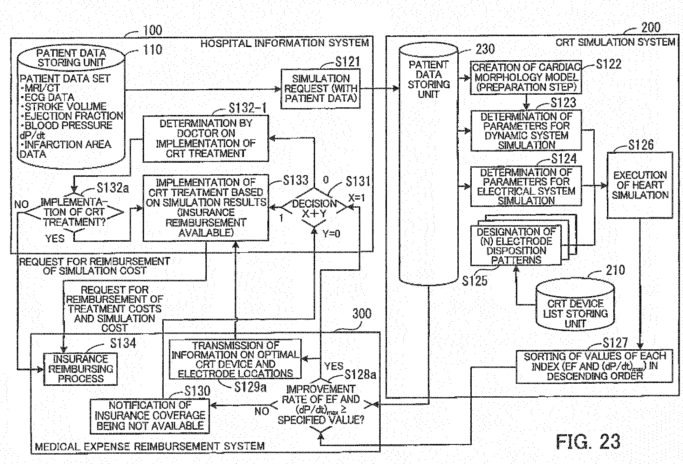

FIG. 23 illustrates an example of a procedure of CRT treatment and medical expense reimbursement according to the seventh embodiment. According to the seventh embodiment, the cost managing unit 130 bifurcates the process according to the determination of the doctor on whether to perform the CRT treatment (step S132a). If determining to perform the CRT treatment, the doctor then carries out the CRT treatment based on the simulation results (step S133). Subsequently, the cost managing unit 130 sends a request for reimbursement of the medical treatment costs and simulation cost to the medical expense reimbursement system 300. On the other hand, if the doctor determines not to perform the CRT device, the cost managing unit 130 sends a request for reimbursement of the simulation cost to the medical expense reimbursement system 300. According to the request from the hospital information system 100, the reimbursement processing unit 310 of the medical expense reimbursement system 300 performs the process of reimbursing the medical treatment costs and/or the simulation cost (step S134).

Thus, according to the seventh embodiment, the simulation results are sent to the medical expense reimbursement system 300, at which the effectiveness of the CRT treatment is determined. Even if the CRT treatment is determined to provide no benefit, the medical expense reimbursement system 300 performs the reimbursement process for the costs of the CRT treatment if the doctor decides to perform the CRT treatment. Thus, even if the CRT simulation results fail to satisfy the improvement rate set in advance by the healthcare insurance company 33, if the CRT treatment is carried out at the discretion of the doctor, the medical treatment costs are reimbursed by the healthcare insurance company 33.

(h) Eighth Embodiment

Next described is an eighth embodiment. According to the eighth embodiment, the healthcare insurance company 33 makes not only the determination of whether the improvement rate achieved by the CRT treatment is predicted to satisfy the specified value, but also the determination on whether to perform the CRT treatment. The eighth embodiment differs from the sixth embodiment in the following point. According to the sixth embodiment, the medical expense reimbursement system 300 determines whether the improvement rate is greater than or equal to the specified value; however, the hospital information system 100 determines whether to perform the CRT treatment. According to the eighth embodiment, the medical expense reimbursement system 300 also makes the determination on whether to perform the CRT treatment.

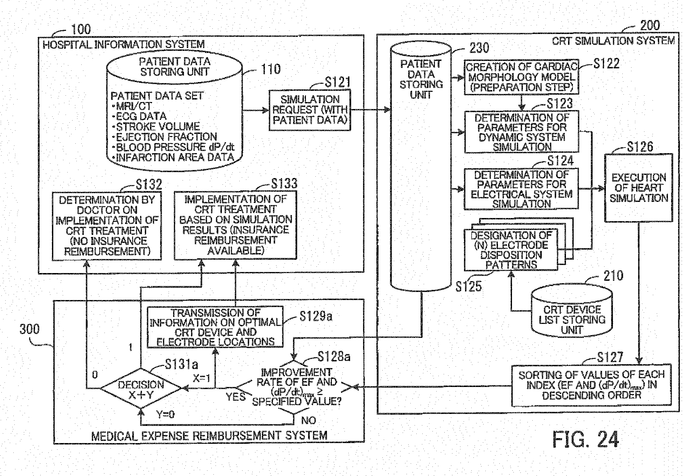

FIG. 24 illustrates an example of a procedure of CRT treatment and medical expense reimbursement according to the eighth embodiment. According to the eighth embodiment, if having determined that the improvement rate is greater than or equal to the specified value (step S128a), the reimbursement processing unit 310 notifies the hospital information system 100 of the optimal CRT device and electrode locations, and also plugs in 1 for the variable X. On the other hand, if having determined that the improvement rate falls below the specified value, the reimbursement processing unit 310 plugs in 0 for the variable Y. Then, the reimbursement processing unit 310 calculates the value obtained by X + Y to thereby determine whether the implementation of the CRT treatment provides benefits (step S131a). If X + Y = 1, the reimbursement processing unit 310 notifies the hospital information system 100 that insurance reimbursement for the CRT treatment will be made. If X + Y = 0, the reimbursement processing unit 310 notifies the hospital information system 100 that no insurance reimbursement will be available for the CRT treatment.

The eighth embodiment allows the healthcare insurance company 33 not only to set in advance the improvement rate providing an indication of insurance reimbursement, but also to determine whether to make insurance reimbursement and inform the hospital 31 of whether the implementation of the CRT treatment provides benefits. This facilitates a quick reimbursement procedure.

(i) Ninth Embodiment

Next described is a ninth embodiment. According to the ninth embodiment, the CRT treatment effectiveness (i.e., whether the implementation of the CRT treatment provides benefits) is determined by both the hospital 31 and the healthcare insurance company 33 (doubly checked). The ninth embodiment differs from the second embodiment in the following point. According to the second embodiment, the healthcare insurance company 33 accepts an application from the hospital 31 for reimbursement of the medical treatment costs straight away. However, according to the ninth embodiment, the medical expense reimbursement system 300 checks the validity of the insurance reimbursement application.

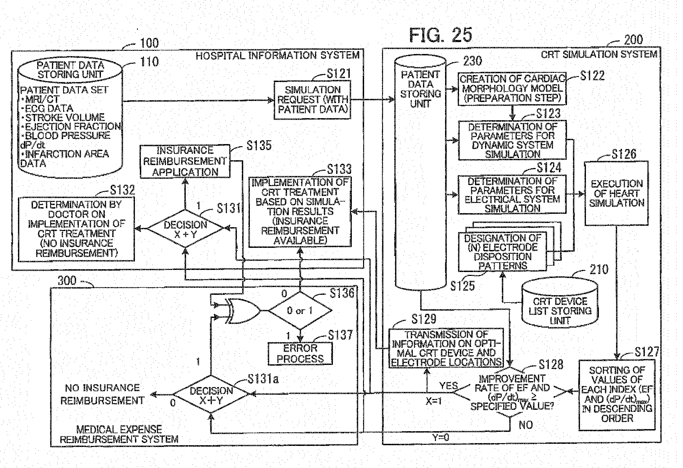

FIG. 25 illustrates an example of a procedure of CRT treatment and medical expense reimbursement according to the ninth embodiment. According to the ninth embodiment, if the improvement rate predicted by the CRT simulation system 200 is determined to be greater than or equal to the specified value (step S128), the determination result is sent to both the hospital information system 100 and the medical expense reimbursement system 300. When the implementation of the CRT treatment is determined to provide benefit, the cost managing unit 130 of the hospital information system 100 sends a value of 1, which represents an insurance reimbursement application, to the medical expense reimbursement system 300 (step S135).

On the other hand, the reimbursement processing unit 310 of the medical expense reimbursement system 300 plugs in 1 for the variable X upon receiving, from the CRT simulation system 200, the determination result indicating that the improvement rate is greater than or equal to the specified value. If receiving a determination result indicating that the improvement rate falls below the specified value from the CRT simulation system 200, the reimbursement processing unit 310 plugs in 0 for the variable Y. Then, the reimbursement processing unit 310 calculates the value obtained by X + Y to thereby determine whether the implementation of the CRT treatment provides benefits (step S131a). If X + Y = 1, the reimbursement processing unit 310 outputs a value of 1, which indicates that insurance reimbursement for the CRT treatment will be made. If X + Y = 0, the reimbursement processing unit 310 outputs a value of 0, which indicates that no insurance reimbursement will be available for the CRT treatment.

Then, when an insurance reimbursement application is issued by the hospital information system 100, the reimbursement processing unit 310 determines whether a value of 1, which indicates that insurance reimbursement for the CRT treatment will be made, has been output (step S136). For example, the reimbursement processing unit 310 calculates an exclusive OR between the value "1" representing an insurance reimbursement application and the result of X + Y obtained by the reimbursement processing unit 310 and then determines whether the calculated result is 0 (both inputs are "1") or 1 (one of the inputs is "0"). In the case where a value of 1 indicating that insurance reimbursement for the CRT treatment will be made has been output (the result of the exclusive OR is "0"), the reimbursement processing unit 310 notifies the hospital information system 100 that the insurance reimbursement for the CRT treatment will be made. In response to the notification, the CRT treatment is carried out at the hospital 31 (step S133).

If a value of 1 indicating that insurance reimbursement for the CRT treatment will be made has not been output (the result of the exclusive OR is "1"), the reimbursement processing unit 310 performs an error process for the insurance reimbursement application (step S137). As the error process, for example, the reimbursement processing unit 310 displays, on a terminal of an administrator, a message indicating that an erroneous insurance reimbursement application has been 4 submitted.

Thus, according to the ninth embodiment, whether the implementation of the CRT treatment provides benefits is determined by both the hospital 31 and the healthcare insurance company 33, and the healthcare insurance company 33 reimburses the medical treatment costs when the determination results of the hospital 31 and the healthcare insurance company 33 agree with each other. Herewith, even if the hospital 31 unilaterally tries to implement the CRT treatment to a patient with a low improvement rate and seeks the insurance reimbursement, the healthcare insurance company 33 is able to run its own check.

(j) Tenth Embodiment

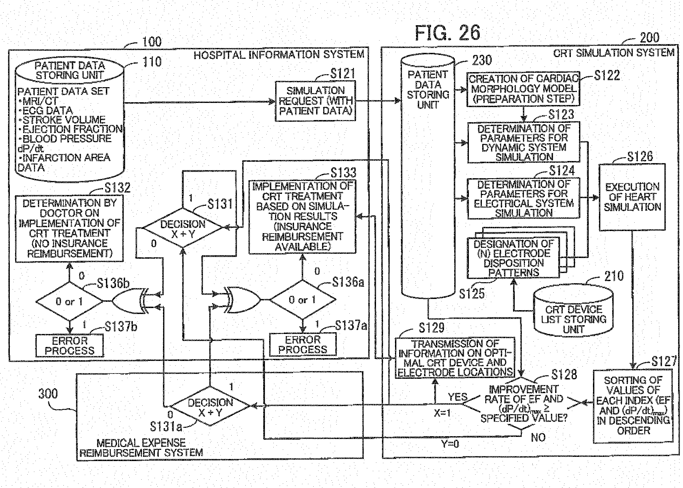

Next described is a tenth embodiment. According to the tenth embodiment, the determination results of the CRT treatment effectiveness on the patient individually obtained by the hospital 31 and the healthcare insurance company 33 are checked against each other by the hospital 31. The tenth embodiment differs from the ninth embodiment in the following points. FIG. 26 illustrates an example of a procedure of CRT treatment and medical expense reimbursement according to the tenth embodiment. According to the tenth embodiment, the determination result of the CRT treatment effectiveness obtained by the reimbursement processing unit 310 of the medical expense reimbursement system 300 (step S131a) is sent to the hospital information system 100. The cost managing unit 130 of the hospital information system 100 checks its own determination result of the CRT treatment effectiveness (step S131) against the determination result sent from the medical expense reimbursement system 300.

For example, the cost managing unit 130 determines whether both the determination results are "1" (the CRT treatment will be effective) (step S136a). If both the determination results are "1", the CRT treatment is carried out with the medical treatment costs covered by insurance (step S133). If one of the decision results is "0", the error process is performed (step S137a).

In addition, the cost managing unit 130 determines whether both the decision results are "0" (the CRT treatment will produce no benefit) (step S136b). If both the decision results are "0", the doctor decides whether to perform the CRT treatment (step S132). If one of the decision results is "1", the error process is performed (step S137b).

Thus, according to the tenth embodiment, the CRT treatment effectiveness on the patient is determined individually by the hospital 31 and the healthcare insurance company 33, and the hospital 31 then checks the decision results against each other. This allows the hospital 31 to prevent incidents where the healthcare insurance company 33 mistakenly treats a patient with a fair improvement rate as a non-responder.

(k) Eleventh Embodiment

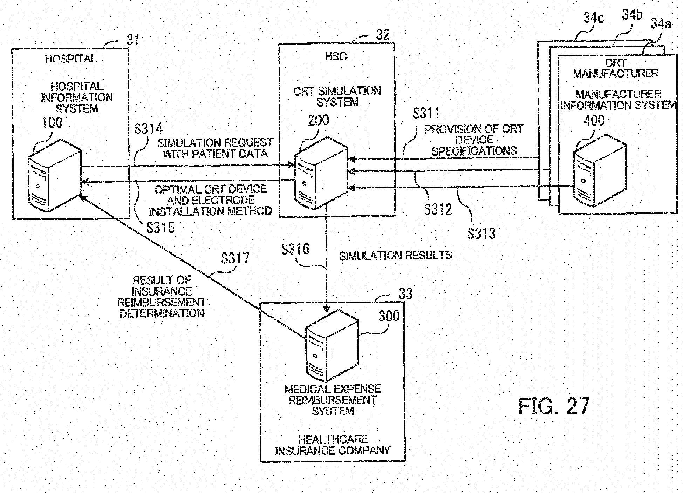

Next described is an eleventh embodiment. According to the eleventh embodiment, CRT simulation is run at the HSC 32 with information on specifications of CRT devices produced by a plurality of manufacturers, thereby improving the accuracy of determining the optimal CRT device for the patient. FIG. 27 illustrates an example of a processing procedure according to the eleventh embodiment. The eleventh embodiment is directed to CRT treatment using one of CRT devices produced by a plurality of CRT device manufacturers 34a, 34b, and 34c. The CRT device manufacturer 34a has a manufacturer information system 400. The manufacturer information system 400 provides the CRT simulation system 200 with specifications of its CRT device (step S311). Each of the remaining CRT device manufacturers 34b and 34c also has a manufacturer information system, which provides the CRT simulation system 200 with specifications of its CRT device (steps S312 and S313).

Later the hospital information system 100 sends a simulation request together with patient data to the CRT simulation system 200 (step S314). The CRT simulation system 200 runs CRT simulation. Then, the CRT simulation system 200 informs the hospital information system 100 of the optimal CRT device and electrode installation method (step S315). The CRT simulation system 200 also informs the medical expense reimbursement system 300 of the simulation results (step S316). Subsequently, the medical expense reimbursement system 300 informs the hospital information system 100 of a result of insurance reimbursement determination (step S317). The hospital 31 performs the CRT treatment with the medical treatment costs being covered according to the insurance reimbursement determination result. Herewith, the hospital 31 is able to receive a recommendation of the most effective CRT device and optimal usage of the CRT device for the patient from the HSC 32.

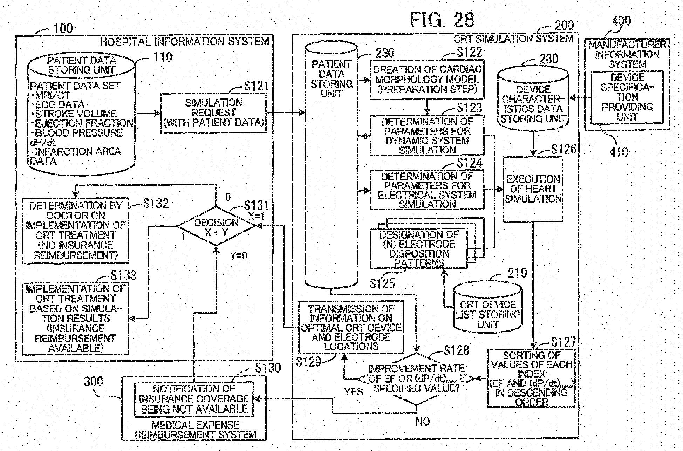

The eleventh embodiment differs from the second embodiment in the following point. FIG. 28 illustrates an example of a procedure of CRT treatment and medical expense reimbursement according to the eleventh embodiment.

According to the eleventh embodiment, the manufacturer information system 400 is provided. The manufacturer information system 400 includes a device specification providing unit 410. The device specification providing unit 410 sends information on CRT device specifications to the CRT simulation system 200. The CRT simulation system 200 is provided with a device characteristics data storing unit 280. The device characteristics data storing unit 280 stores therein specification information of a CRT device, provided by the manufacturer information system 400. With reference to the device characteristics data storing unit 280, the heart simulator 270 sets parameters according to each simulation-target CRT device and runs heart simulation (step S126).

According to the eleventh embodiment, the CRT simulation system 200 holds, as a database, specifications of all CRT devices produced by individual CRT device manufacturers. Therefore, it is possible to give the hospital 31 and the healthcare insurance company 33 a recommendation of the optimal CRT device selected from the registered CRT devices and the optimal usage of the selected CRT device.

(1) Twelfth Embodiment

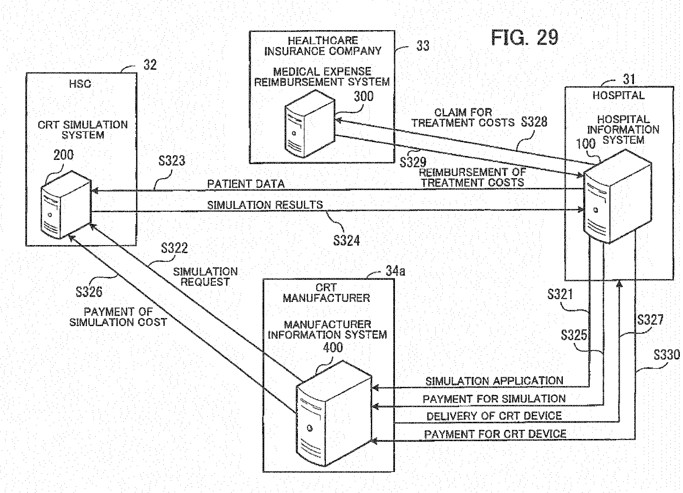

Next described is a twelfth embodiment. The twelfth embodiment is directed to the CRT treatment procedure undertaken at the initiative of a CRT device manufacturer. FIG. 29 illustrates an example of a processing procedure according to the twelfth embodiment. In the case where the doctor determines that the CRT treatment is effective to treat the patient's conditions, the hospital information system 100 sends a CRT simulation application together with the patient data to the manufacturer information system 400 of the CRT device manufacturer 34a (step S321). The manufacturer information system 400 sends a simulation request together with the patient data to the CRT simulation system 200 of the HSC 32 (step S322). The patient data may be sent directly from the hospital information system 100 to the CRT simulation system 200 (step S323).

The CRT simulation system 200 of the HSC 32 runs CRT simulation and then sends results of the simulation to the hospital 31 (step S324). The simulation results may be sent to the hospital 31 via the CRT device manufacturer 34a. Subsequently, using the hospital information system 100, the hospital 31 arranges for payment of the simulation cost to the CRT device manufacturer 34a (step S325). For example, the hospital information system 100 sends money to cover the simulation cost to a bank account of the CRT device manufacturer 34a and then notifies the manufacturer information system 400 of the transfer result. Using the manufacturer information system 400, the CRT device manufacturer 34a arranges for payment of the simulation cost to the HSC 32 (step S326). For example, the manufacturer information system 400 sends money to cover the simulation cost to a bank account of the HSC 32 and then notifies the CRT simulation system 200 of the transfer result.

At the hospital 31, the hospital information system 100 determines the effectiveness of the CRT treatment implementation based on the simulation results. If the CRT treatment is determined to be implemented, the hospital 31 receives a delivery of the CRT device from the CRT device manufacturer 34a (step S327). Then, at the hospital 31, the doctor performs a surgical procedure to implant the delivered CRT device in the patient. Subsequently, using the hospital information system 100, the hospital 31 makes a claim for the medical treatment costs (including the simulation cost) against the healthcare insurance company 33 (step S328). For example, the hospital information system 100 sends an invoice for the medical treatment costs to the medical expense reimbursement system 300. At the healthcare insurance company 33, the medical expense reimbursement system 300 performs the process of reimbursing the medical treatment costs (step S329). Using the hospital information system 100, the hospital 31 arranges for payment for the CRT device (including the simulation cost) to the CRT device manufacturer 34a (step S330).

Such a system allows the CRT device manufacturer to play a leading role in recommending the optimal CRT device while making efficient use of the CRT simulation. As a result, the patient is able to receive CRT treatment using the optimal CRT device for the patient, thus promising a significant treatment effect on the patient.

(m) Thirteenth Embodiment

Next described is a thirteenth embodiment. The thirteenth embodiment is directed to, when there are a plurality of manufacturers providing CRT devices, running CRT simulation in which characteristics of the CRT device of each manufacturer are reflected to thereby select the optimal CRT device for the patient. That is, CRT devices are not necessarily provided by only one manufacturer; a plurality of manufacturers produce CRT devices all having their distinctive characteristics and provide them to the hospital 31. In view of this, according to the thirteenth embodiment, the HSC 32 selects the optimal CRT device for the patient based on a request from the hospital 31 and makes a recommendation on the optimal usage of the CRT device to the hospital 31.

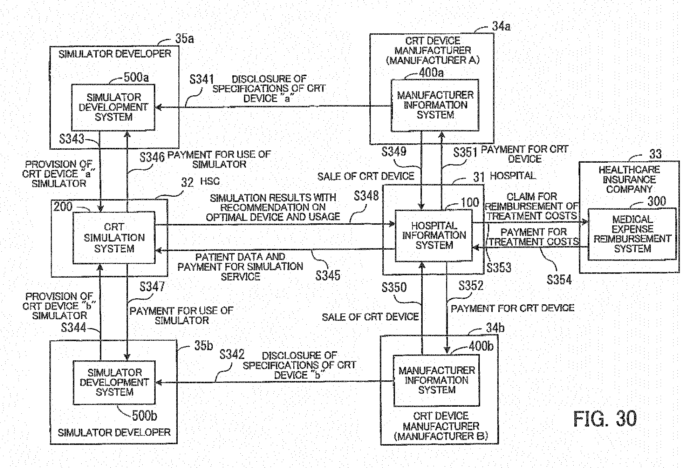

FIG. 30 illustrates an example of a processing procedure according to the thirteenth embodiment. According to the thirteenth embodiment, the HSC 32 is preliminarily provided, from each of the CRT device manufacturers 34a and 34b, with adequate disclosure of specifications unique to the manufacturer, and then prepares simulation for determining the optimal usage of a CRT device of the manufacturer and makes it executable. In this regard, specifications of a CRT device "a" distributed by the CRT device manufacturer 34a are disclosed to a simulator developer 35a (step S341). Similarly, specifications of a CRT device "b" distributed by the CRT device manufacturer 34b are disclosed to a simulator developer 35b (step S342). For example, information of CRT device specifications is sent from manufacturer information systems 400a and 400b to simulator development systems 500a and 500b, respectively, of the individual simulator developers 35a and 35b.

The simulator developer 35a develops a simulator designed for the CRT device "a" and provides it to the HSC 32 (step S343). The simulator developer 35b develops a simulator designed for the CRT device "b" and provides it to the HSC 32 (step S344). For example, each of the simulator development systems 500a and 500b of the simulator developers 35a and 35b, respectively, sends a CRT simulation program to the CRT simulation system 200 of the HSC 32. Later when the hospital 31 makes a simulation request to the HSC 32, the hospital information system 100 sends the patient data to the CRT simulation system 200 and arranges for payment for the CRT simulation service to the CRT simulation system 200 (step S345). The CRT simulation system 200 runs CRT simulation based on the patient data using the simulators designed for the individual CRT devices. Then, the CRT simulation system 200 arranges for payment for the use of the simulator to each of the simulator developers 35a and 35b (steps S346 and S347).

After completing the CRT simulation, the CRT simulation system 200 sends results of the simulation to the hospital information system 100 (step S348). The simulation results include a recommendation on the optimal CRT device and optimal method of how to apply the CRT device (for example, electrode installation locations in the case where four electrodes are provided on a coronary sinus lead). The hospital 31 places an order for the optimal CRT device based on the CRT simulation results. The simulation results also include, with respect to each CRT device, a combination pattern of electrode locations predicted to produce the greatest improvement in the cardiac function of the patient and data of the cardiac function (such as ECG data, ejection fraction, $(dP/dt)_{max}$, and data visualizing ventricular motion) associated with each of all the combination patterns. A CRT device manufacturer having received the order (the CRT device manufacturer 34a or 34b) sells the ordered CRT device to the hospital 31 (step S349 or S350). Using the hospital information system 100, the hospital 31 arranges for payment for the CRT device to the CRT device manufacturer (the CRT device manufacturer 34a or 34b) (step S351 or S352). Then, the hospital 31 implements CRT treatment using a method that is based on the CRT simulation results. Subsequently, the hospital 31 makes a claim for reimbursement of the medical treatment costs including the simulation cost against the healthcare insurance company 33, using the hospital information system 100 (step S353). At the healthcare insurance company 33, the medical expense reimbursement system 300 arranges for payment for the medical treatment costs (step S354).

In the above-described manner, it is possible to select the optimal CRT device amongst CRT devices produced and distributed by a plurality of CRT device manufacturers 34a and 34b and cover the cost of simulation used to make the selection by medical care insurance.

(n) Fourteenth Embodiment

Next described is a fourteenth embodiment. According to the fourteenth embodiment, not the hospital 31 but the healthcare insurance company 33 selects in advance the optimal CRT device amongst CRT devices of a plurality of CRT device manufacturers. The healthcare insurance company 33 makes the selection in consideration of the cost-effectiveness for each CRT device, which stimulates price competition among the CRT device manufacturers.

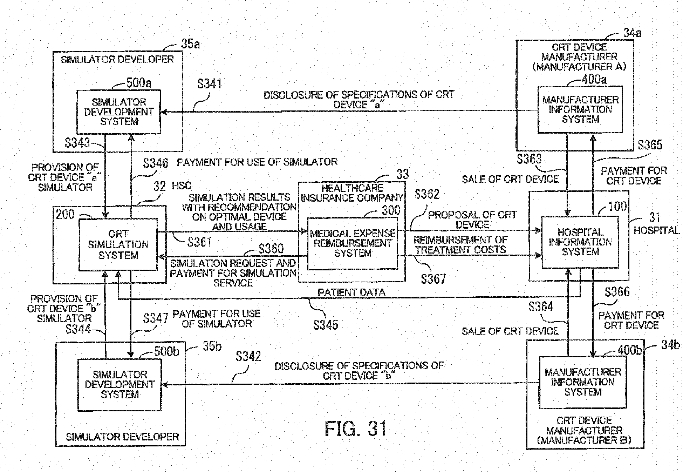

The fourteenth embodiment differs from the thirteenth embodiment in the following points. FIG. 31 illustrates an example of a processing procedure according to the fourteenth embodiment. According to the fourteenth embodiment, the medical expense reimbursement system 300 of the healthcare insurance company 33 makes a simulation request and arranges for payment for the CRT simulation service to the HSC 32 (step S360). Then, the CRT simulation system 200 sends, to the medical expense reimbursement system 300, results of the simulation and information on the optimal CRT device and optimal usage of the CRT device (step S361). The medical expense reimbursement system 300 then makes an assessment by taking into account the prices of CRT devices in addition to the improvement rate of, for example, the ejection fraction of each CRT device indicated by the simulation results, to thereby determine the optimal CRT device. Then, the medical expense reimbursement system 300 gives the hospital information system 100 a recommendation of the optimal CRT device (step S362). Subsequently, as in steps S349 to S352 of the thirteenth embodiment, the CRT device sale and the payment for the CRT device (steps S363 to S366) are made. In response, the medical expense reimbursement system 300 processes procedures to reimburse the medical treatment costs (step S367).

In the above-described manner, by allowing the healthcare insurance company 33 to select the optimal CRT device, the prices of CRT devices are also incorporated into the consideration, which leads to CRT treatment using a cost-effective CRT device and thus enables a reduction in medical treatment costs.

### (o) Fifteenth Embodiment

Next described is a fifteenth embodiment. According to the fifteenth embodiment, the doctor determines the optimal CRT device. The hospital information system 100 determines in advance the optimal CRT device for the patient and asks a manufacturer of the determined CRT device to run CRT simulation. Alternatively, the hospital information system 100 may ask a plurality of CRT device manufacturers to submit simulation results and then determine the optimal CRT device for the patient based on the results.

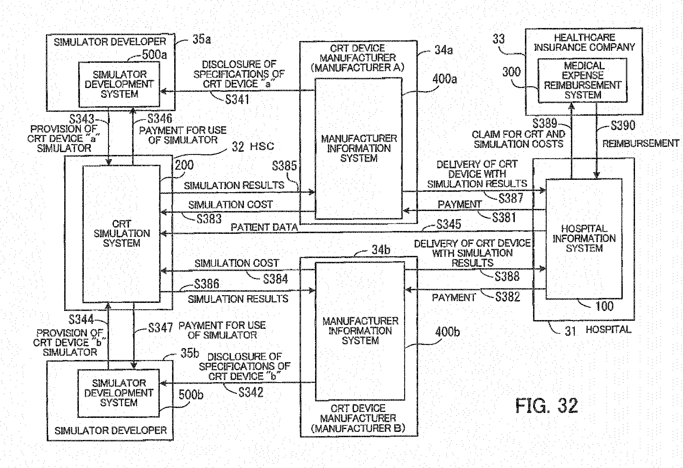

The fifteenth embodiment differs from the thirteenth embodiment in the following points. FIG. 32 illustrates an example of a processing procedure according to the fifteenth embodiment. According to the fifteenth embodiment, the hospital information system 100 determines the optimal CRT device amongst the CRT devices produced by a plurality of CRT device manufacturers 34a and 34b. Then, the hospital information system 100 arranges for payment to a CRT device manufacturer producing the determined CRT device (step S381 or S382) and requests the CRT device manufacturer to deliver the CRT device with simulation results. In response to the request, the manufacturer information system of the CRT device manufacturer requests the CRT simulation system 200 to run the CRT simulation by paying the simulation cost (step S383 or step S384). The CRT simulation system 200 runs the CRT simulation using a simulator designed for the CRT device and then sends results of the simulation to the manufacturer information system (step S385 or S386). The manufacturer information system delivers the CRT device with the simulation results to the hospital information system 100 (step S387 or S388). At the hospital 31, implantation of the delivered CRT device is carried out, and the hospital information system 100 makes a claim for the CRT cost and the simulation cost against the medical expense reimbursement system 300 (step S389). In response, the medical expense reimbursement system 300 arranges for payment for the expenses (step S390). Thus, the doctor selects an appropriate CRT device for the patient while interpreting the simulation results base on the medical expertise.

### (p) Sixteenth Embodiment

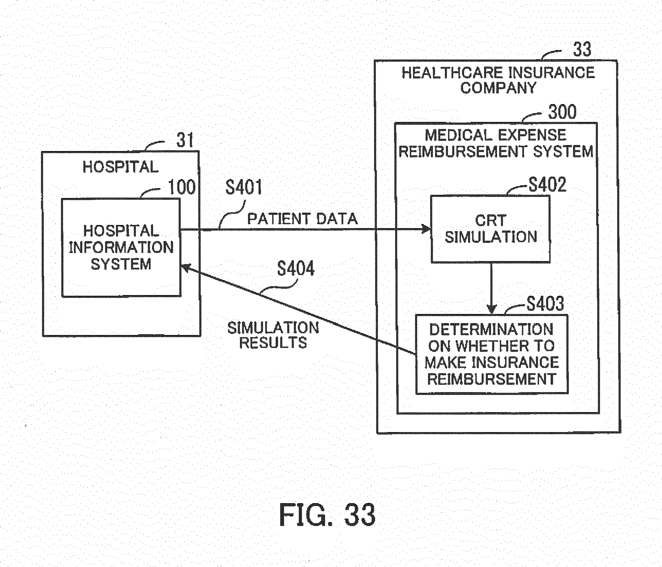

Next described is a sixteenth embodiment. The sixteenth embodiment is directed to performing the CRT simulation inside the healthcare insurance company 33. FIG. 33 illustrates an example of a processing procedure according to the sixteenth embodiment. The hospital information system 100 sends the patient data to the medical expense reimbursement system 300 (step S401). The medical expense reimbursement system 300 runs the CRT simulation based on the patient data (step S402) and determines whether to reimburse the medical treatment costs by insurance (step S403). Then, the medical expense reimbursement system 300 sends, to the hospital information system 100, results of the simulation including information on whether to reimburse the medical treatment costs (step S404). Thus, performing the CRT simulation using the inner system of the healthcare insurance company 33 facilitates quick decision-making on whether to make insurance reimbursement.

According to an embodiement of one aspect of the invention, it is possible to reduce medical expenses.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

## Claims

1. A biomedical simulation system comprising:

    biomedical information storing means (11a) for storing biomedical information specific to a patient;
    simulation execution determining means (11b) for determining, based on the biomedical information, whether to perform biomedical simulation;
    model generating means (12a) for generating a biomedical model of a heart of the patient based on the biomedical information when the simulation execution determining means (11b) has determined to perform the biomedical simulation;
    simulation executing means (12b) for executing, based on the biomedical model generated by the model generating means (12a), the biomedical simulation of post-operative conditions of the heart following an operation to implant a cardiac resynchronization therapy device performed on the patient, for each of a plurality of electrode disposition patterns associated with implantation of the cardiac resynchronization therapy device;

improvement rate determining means (12c) for determining whether results of the biomedical simulation executed by the simulation executing means (12b) meet a predetermined improvement rate associated with symptoms;

treatment expense reimbursing means (13a) for determining, when the improvement rate determining means (12c) has determined that the results of the biomedical simulation do not meet the predetermined improvement rate, not to reimburse medical treatment costs for the operation of the patient; and

operation decision-making means (11c) for deciding to perform the operation on the patient when the improvement rate determining means (12c) has determined that the results of the biomedical simulation meet the predetermined improvement rate.

2.  The biomedical simulation system according to claim 1, wherein:

the biomedical information storing means (11a), the simulation execution determining means (11b), and the operation decision-making means (11c) are installed at a medical facility where the operation is performed on the patient,

the model generating means (12a), the simulation executing means (12b), and the improvement rate determining means (12c) are installed at a simulation center, and

the treatment expense reimbursing means (13a) is installed at a health insurance company for reimbursing the medical treatment costs.

3.  The biomedical simulation system according to claim 1 or 2, wherein:

the biomedical information storing means (11a) and the simulation execution determining means (11b) are installed at a medical facility where the operation is performed on the patient,

the model generating means (12a), the simulation executing means (12b), and the improvement rate determining means (12c) are installed at a simulation center, and

the operation decision-making means (11c) and the treatment expense reimbursing means (13a) are installed at a health insurance company for reimbursing the medical treatment costs.

4.  The biomedical simulation system according to any of claims 1 to 3, wherein:

the biomedical information storing means (11a), the simulation execution determining means (11b), and the operation decision-making means (11c) are installed at a medical facility where the operation is performed on the patient,

the model generating means (12a) and the simulation executing means (12b) are installed at a simulation center, and

the improvement rate determining means (12c) and the treatment expense reimbursing means (13a) are installed at a health insurance company for reimbursing the medical treatment costs.

5.  The biomedical simulation system according to any one of claims 1 to 4, wherein:

the biomedical information storing means (11a) and the simulation execution determining means (11b) are installed at a medical facility where the operation is performed on the patient,

the model generating means (12a) and the simulation executing means (12b) are installed at a simulation center, and

the improvement rate determining means (12c), the operation decision-making means (11c), and the treatment expense reimbursing means (13a) are installed at a health insurance company for reimbursing the medical treatment costs.

6.  The biomedical simulation system according to any one of claims 1 to 5, wherein:

the operation decision-making means (11c) includes first decision-making means for outputting an insurance reimbursement application when the improvement rate determining means (12c) has determined that the results of the biomedical simulation meet the predetermined improvement rate, and second decision-making means for deciding to perform the operation on the patient when the improvement rate determining means (12c) has determined that the results of the biomedical simulation meet the predetermined improvement rate and, then, the first decision-making unit has output the insurance reimbursement application,

the biomedical information storing means (11a), the simulation execution determining means (11b), and the first decision-making means are installed at a medical facility where the operation is performed on the patient, the model generating means (12a), the simulation executing means (12b), and the improvement rate determining means (12c) are installed at a simulation center, and

the second decision-making means and the treatment expense reimbursing means (13a) are installed at a health insurance company for reimbursing the medical treatment costs.

7. The biomedical simulation system according to any one of claims 1 to 6, wherein:

the treatment expense reimbursing means (13a) determines to reimburse the medical treatment costs when the improvement rate determining means (12c) has determined that the results of the biomedical simulation meet the predetermined improvement rate, and

the operation decision-making means (11c) decides to perform the operation on the patient when the improvement rate determining means (12c) has determined that the results of the biomedical simulation meet the predetermined improvement rate and, then, the operation decision-making means (11c) obtains confirmation of the treatment expense reimbursing means (13a) having determined to reimburse the medical treatment costs.

8. The biomedical simulation system according to any one of claims 1 to 7, wherein:
the treatment expense reimbursing means (13a) reimburses the medical treatment costs and costs for executing the biomedical simulation when the operation is performed on the patient, and reimburses the costs for executing the biomedical simulation when the operation is not performed on the patient.

9. The biomedical simulation system according to any one of claims 1 to 8, further comprising:

device characteristics data storing means (280) for storing device characteristics data indicating characteristics of the cardiac resynchronization therapy device to be used in the operation of the patient,
wherein the simulation executing means (12b) refers to the device characteristics data to execute the biomedical simulation in which the characteristics of the cardiac resynchronization therapy device are reflected.

10. The biomedical simulation system according to any one of claims 1 to 9, wherein:
the improvement rate determining means (12c) selects, amongst the applicable operative procedures, an operative procedure with the results of the biomedical simulation showing a highest improvement rate, and determines whether the highest improvement rate meets the predetermined improvement rate.

11. The biomedical simulation system according to any one of claims 1 to 10, wherein:
the improvement rate determining means (12c) makes the determination associated with the predetermined improvement rate based on an evaluation value which is a sum of values, each of the values being obtained by multiplying an improvement rate of each of a plurality of indexes, obtained for each electrode disposition pattern, by a weight value corresponding to the index, the improvement rate of each of the indexes being included in the results obtained from the biomedical simulation executed for the individual operative procedures.

12. The biomedical simulation system according to claim 11, wherein:
the weight value corresponding to each of the indexes is set in advance from a medical perspective.

13. A biomedical simulation method executed by a biomedical simulation system including at least one computer, the biomedical simulation method comprising:

determining, based on biomedical information specific to a patient, whether to perform biomedical simulation;
generating a biomedical model of a heart of the patient based on the biomedical information when having determined to perform the biomedical simulation;
executing, based on the biomedical model, the biomedical simulation of post-operative conditions of the heart following an operation to implant a cardiac resynchronization therapy device performed on the patient for each of a plurality of electrode disposition patterns associated with implantation of the cardiac resynchronization therapy device;
determining whether results of the biomedical simulation meet a predetermined improvement rate associated with symptoms;
determining, when the results of the biomedical simulation do not meet the predetermined improvement rate, not to reimburse medical treatment costs for the operation of the patient; and
deciding to perform the operation on the patient when the results of the biomedical simulation meet the predetermined improvement rate.

14. A computer program which, when executed by a biomedical simulation system including at least one computer, causes the biomedical simulation system to perform the method according to claim 13.

**Patentansprüche**

1. System zur biomedizinischen Simulation, umfassend:

Mittel zur biomedizinischen Informationsspeicherung (11a) zum Speichern biomedizinischer Informationen, die für einen Patienten spezifisch sind;

Mittel zur Simulationsausführungsbestimmung (11b) zum Bestimmen, auf der Grundlage der biomedizinischen Informationen, ob eine biomedizinische Simulation ausgeführt werden soll;

Mittel zur Modellerstellung (12a) zum Erstellen eines biomedizinischen Modells eines Patientenherzens auf der Grundlage der biomedizinischen Informationen, wenn das Mittel zur Simulationsausführungsbestimmung (11b) bestimmt hat, die biomedizinische Simulation auszuführen;

Mittel zur Simulationsausführung (12b) zum Ausführen, auf der Grundlage des biomedizinischen Modells, das von dem Mittel zur Modellerstellung (12a) erstellt wurde, der biomedizinischen Simulation von postoperativen Zuständen des Herzens nach einer Operation zum Implantieren eines Gerätes zur kardialen Resynchronisationstherapie, die am Patienten durchgeführt ist, für jeweils jede von mehreren Elektrodenanordnungsmustern in Verbindung mit der Implantation eines Gerätes zur kardialen Resynchronisationstherapie;

Mittel zur Verbesserungsratenbestimmung (12c) zum Bestimmen, ob Ergebnisse der biomedizinischen Simulation, die von dem Mittel zur Simulationsausführung (12b) ausgeführt ist, eine vorbestimmte Verbesserungsrate im Zusammenhang mit Symptomen erfüllen;

Mittel zur Behandlungskostenerstattung (13a) zum Bestimmen, wenn das Mittel zur Verbesserungsratenbestimmung (12c) bestimmt hat, dass die Ergebnisse der biomedizinischen Simulation nicht die vorbestimmte Verbesserungsrate erfüllen, dass die medizinische Behandlungskosten für die Operation des Patienten nicht erstattet werden; und

Mittel zur Operationsentscheidung (11c) zum Treffen der Entscheidung, die Operation am Patienten durchzuführen, wenn das Mittel zur Verbesserungsratenbestimmung (12c) bestimmt hat, dass die Ergebnisse der biomedizinischen Simulation die vorbestimmte Verbesserungsrate erfüllen.

2. System zur biomedizinischen Simulation nach Anspruch 1, wobei:

das Mittel zur biomedizinischen Informationsspeicherung (11a), das Mittel zur Simulationsausführungsbestimmung (11b) und das Mittel zur Operationsentscheidung (11c) bei einer medizinischen Einrichtung, in der die Operation am Patienten durchgeführt wird, installiert sind,

das Mittel zur Modellerstellung (12a), das Mittel zur Simulationsausführung (12b) und das Mittel zur Verbesserungsratenbestimmung (12c) bei einem Simulationszentrum installiert sind, und

das Mittel zur Behandlungskostenerstattung (13a) bei einem Krankenversicherungsunternehmen zur Erstattung medizinischer Behandlungskosten installiert ist.

3. System zur biomedizinischen Simulation nach Anspruch 1 oder 2, wobei:

das Mittel zur biomedizinischen Informationsspeicherung (11a) und das Mittel zur Simulationsausführungsbestimmung (11b) bei einer medizinischen Einrichtung, in der die Operation am Patienten durchgeführt wird, installiert sind,

das Mittel zur Modellerstellung (12a), das Mittel zur Simulationsausführung (12b) und das Mittel zur Verbesserungsratenbestimmung (12c) bei einem Simulationszentrum installiert sind, und

das Mittel zur Operationsentscheidung (11c) und das Mittel zur Behandlungskostenerstattung (13a) bei einem Krankenversicherungsunternehmen zum Erstatten der medizinischen Behandlungskosten installiert sind.

4. System zur biomedizinischen Simulation nach einem der Ansprüche 1 bis 3, wobei:

das Mittel zur biomedizinischen Informationsspeicherung (11a), das Mittel zur Simulationsausführungsbestimmung (11b) und das Mittel zur Operationsentscheidung (11c) bei einer medizinischen Einrichtung, in der die Operation am Patienten durchgeführt wird, installiert sind,

das Mittel zur Modellerstellung (12a) und das Mittel zur Simulationsausführung (12b) bei einem Simulationszentrum installiert sind, und

das Mittel zur Verbesserungsratenbestimmung (12c) und das Mittel zur Behandlungskostenerstattung (13a) bei einem Krankenversicherungsunternehmen zum Erstatten der medizinischen Behandlungskosten installiert sind.

5. System zur biomedizinischen Simulation nach einem der Ansprüche von 1 bis 4, wobei:

das Mittel zur biomedizinischen Informationsspeicherung (11a) und das Mittel zur Simulationsausführungsbestimmung (11b) bei einer medizinischen Einrichtung, in der die Operation am Patienten durchgeführt wird, installiert sind,

das Mittel zur Modellerstellung (12a) und das Mittel zur Simulationsausführung (12b) bei einem Simulationszentrum installiert sind, und

das Mittel zur Verbesserungsratenbestimmung (12c), das Mittel zur Operationsentscheidung (11c) und das Mittel zur Behandlungskostenerstattung (13a) bei einem Krankenversicherungsunternehmen zum Erstatten der medizinischen Behandlungskosten installiert sind.

6. System zur biomedizinischen Simulation nach einem der Ansprüche von 1 bis 5, wobei:

das Mittel zur Operationsentscheidung (11c) umfasst: erstes Entscheidungsmittel zur Ausgabe eines Versicherungserstattungsantrags, wenn das Mittel zur Verbesserungsratenbestimmung (12c) bestimmt hat, dass die Ergebnisse der biomedizinischen Simulation die vorbestimmte Verbesserungsrate erfüllen, und zweites Entscheidungsmittel zum Treffen der Entscheidung, die Operation am Patienten durchzuführen, wenn das Mittel zur Verbesserungsratenbestimmung (12c) bestimmt hat, dass die Ergebnisse der biomedizinischen Simulation die vorbestimmte Verbesserungsrate erfüllen, und, anschließend, die erste Entscheidungseinheit den Versicherungserstattungsantrag ausgegeben hat,

das Mittel zur biomedizinischen Informationsspeicherung (11a), das Mittel zur Simulationsausführungsbestimmung (11b) und das erste Entscheidungsmittel bei einer medizinischen Einrichtung, in der die Operation am Patienten durchgeführt wird, installiert sind, das Mittel zur Modellerstellung (12a), das Mittel zur Simulationsausführung (12b) und das Mittel zur Verbesserungsratenbestimmung (12c) bei einem Simulationszentrum installiert sind, und

das zweite Entscheidungsmittel und das Mittel zur Behandlungskostenerstattung (13a) bei einem Krankenversicherungsunternehmen zum Erstatten der medizinischen Behandlungskosten installiert sind.

7. System zur biomedizinischen Simulation nach einem der Ansprüche von 1 bis 6, wobei:

das Mittel zur Behandlungskostenerstattung (13a) bestimmt, die medizinischen Behandlungskosten zu erstatten, wenn das Mittel zur Verbesserungsratenbestimmung (12c) bestimmt hat, dass die Ergebnisse der biomedizinischen Simulation die vorbestimmte Verbesserungsrate erfüllen, und

das Mittel zur Operationsentscheidung (11c) entscheidet, die Operation am Patienten durchzuführen, wenn das Mittel zur Verbesserungsratenbestimmung (12c) bestimmt hat, dass die Ergebnisse der biomedizinischen Simulation die vorbestimmte Verbesserungsrate erfüllen und, anschließend, das Mittel zur Operationsentscheidung (11c) Bestätigung darüber erhält,

dass das Mittel zur Behandlungskostenerstattung (13a) bestimmt hat, die medizinischen Behandlungskosten zu erstatten.

8. System zur biomedizinischen Simulation nach einem der Ansprüche von 1 bis 7, wobei:

das Mittel zur Behandlungskostenerstattung (13a) die medizinischen Behandlungskosten und

die Ausführungskosten der biomedizinischen Simulation erstattet, wenn die Operation am Patienten durchgeführt ist, und die Ausführungskosten der biomedizinischen Simulation erstattet, wenn die Operation am Patienten nicht durchgeführt ist.

9. System zur biomedizinischen Simulation nach einem der Ansprüche von 1 bis 8, ferner umfassend:

Geräteeigenschaftsdatenspeichermittel (280) zum Speichern von Geräteeigenschaftsdaten, die auf Eigenschaften des Geräts zur kardialen Resynchronisationstherapie hinweisen, welches bei der Operation des Patienten verwendet werden soll,

wobei sich das Mittel zur Simulationsausführung (12b) auf die Geräteeigenschaftsdaten bezieht, um die biomedizinische Simulation, in der sich die Eigenschaften des Geräts zur kardialen Resynchronisationstherapie widerspiegeln, auszuführen.

10. System zur biomedizinischen Simulation nach einem der Ansprüche von 1 bis 9, wobei:
das Mittel zur Verbesserungsratenbestimmung (12c) unter den anwendbaren operativen Verfahren ein operatives Verfahren auswählt, bei dem die Ergebnisse der biomedizinischen Simulation eine höchste Verbesserungsrate aufweisen, und bestimmt, ob die höchste Verbesserungsrate die vorbestimmte Verbesserungsrate erfüllt.

11. System zur biomedizinischen Simulation nach einem der Ansprüche von 1 bis 10, wobei:
das Mittel zur Verbesserungsratenbestimmung (12c) die Bestimmung, die mit der vorbestimmten Verbesserungsrate im Zusammenhang steht, auf der Grundlage eines Beurteilungswertes vornimmt, der eine Summe von Werten ist, wobei jeder der Werte erzielt ist, in dem eine Verbesserungsrate von jedem von mehreren Indizes, die für jedes Elektrodenanordnungsmuster erhalten wurden, mit einem Gewichtswert, der dem Index entspricht, multipliziert wird, wobei die Verbesserungsrate von jedem der Indizes in den Ergebnissen einbezogen ist, die aus der biomedizinischen Simulation, die für die einzelnen operativen Verfahren ausgeführt wurde, erhalten wurden.

12. System zur biomedizinischen Simulation nach Anspruch 11, wobei:
der Gewichtswert, der jedem der Indizes entspricht, vorab aus medizinischer Sicht festgelegt ist.

13. Verfahren zur biomedizinischen Simulation, das von einem System zur biomedizinischen Simulation, welches mindestens einen Computer umfasst, ausgeführt wird, wobei das Verfahren zur biomedizinischen Simulation umfasst:

Bestimmen, auf der Grundlage biomedizinischer Informationen, die für einen Patienten spezifisch sind, ob die biomedizinische Simulation ausgeführt werden soll;
Erstellen eines biomedizinischen Modells eines Patientenherzens auf der Grundlage der biomedizinischen Informationen, wenn bestimmt worden ist, die biomedizinische Simulation auszuführen;
Ausführen, auf der Grundlage des biomedizinischen Modells, der biomedizinischen Simulation von postoperativen Zuständen des Herzens nach einer Operation zum Implantieren eines Gerätes zur kardialen Resynchronisationstherapie, die am Patienten durchgeführt ist, für jeweils jede von mehreren Elektrodenanordnungsmustern in Verbindung mit der Implantation eines Gerätes zur kardialen Resynchronisationstherapie;
Bestimmen, wenn die Ergebnisse der biomedizinischen Simulation nicht die vorbestimmte Verbesserungsrate erfüllen, die medizinischen Behandlungskosten für die Operation des Patienten nicht zu erstatten; und
Entscheiden, die Operation am Patienten durchzuführen, wenn die Ergebnisse der biomedizinischen Simulation die vorbestimmte Verbesserungsrate erfüllen.

14. Computerprogramm, welches bei Ausführung auf einem System zur biomedizinischen Simulation, das mindestens einen Computer umfasst, das System zur biomedizinischen Simulation veranlasst, das Verfahren nach Anspruch 13 durchzuführen.

**Revendications**

1. Système de simulation biomédicale comprenant :

des moyens de stockage d'informations biomédicales (11a) pour stocker des informations biomédicales spécifiques à un patient ;
des moyens de détermination d'exécution de simulation (11b) pour déterminer, sur la base des informations biomédicales, s'il faut effectuer une simulation biomédicale ;
des moyens de génération de modèle (12a) pour générer un modèle biomédical d'un cœur du patient sur la base des informations biomédicales lorsque les moyens de détermination d'exécution de simulation (11b) ont déterminé d'effectuer la simulation biomédicale ;
des moyens d'exécution de simulation (12b) pour exécuter, sur la base du modèle biomédical généré par les moyens de génération de modèle (12a), la simulation biomédicale de conditions postopératoires du cœur suite à une opération d'implantation d'un dispositif de thérapie de resynchronisation cardiaque effectuée sur le patient, pour chacun d'une pluralité de motifs de disposition d'électrodes associés à l'implantation du dispositif de thérapie de resynchronisation cardiaque ;
des moyens de détermination de taux d'amélioration (12c) pour déterminer si des résultats de la simulation biomédicale exécutée par les moyens d'exécution de simulation (12b) satisfont à un taux d'amélioration prédéterminé associé aux symptômes ;

des moyens de remboursement de frais de traitement (13a) pour déterminer, lorsque les moyens de détermination de taux d'amélioration (12c) ont déterminé que les résultats de la simulation biomédicale ne satisfont pas au taux d'amélioration prédéterminé, de ne pas rembourser les coûts de traitement médical pour l'opération du patient ; et

des moyens de prise de décision d'opération (11c) pour décider d'effectuer l'opération sur le patient lorsque les moyens de détermination de taux d'amélioration (12c) ont déterminé que les résultats de la simulation biomédicale satisfont aux taux d'amélioration prédéterminé.

2. Système de simulation biomédicale selon la revendication 1, dans lequel :

les moyens de stockage d'informations biomédicales (11a), les moyens de détermination d'exécution de simulation (11b) et les moyens de prise de décision d'opération (11c) sont installés dans un établissement médical où l'opération est effectuée sur le patient,

les moyens de génération de modèle (12a), les moyens d'exécution de simulation (12b) et les moyens de détermination de taux d'amélioration (12c) sont installés dans un centre de simulation, et

les moyens de remboursement de frais de traitement (13a) sont installés dans une compagnie d'assurance maladie pour rembourser les coûts de traitement médical.

3. Système de simulation biomédicale selon la revendication 1 ou 2, dans lequel :

les moyens de stockage d'informations biomédicales (11a) et les moyens de détermination d'exécution de simulation (11b) sont installés dans un établissement médical où l'opération est effectuée sur le patient,

les moyens de génération de modèle (12a), les moyens d'exécution de simulation (12b) et les moyens de détermination de taux d'amélioration (12c) sont installés dans un centre de simulation, et

les moyens de prise de décision d'opération (11c) et les moyens de remboursement de frais de traitement (13a) sont installés dans une compagnie d'assurance maladie pour rembourser les coûts de traitement médical.

4. Système de simulation biomédicale selon l'une quelconque des revendications 1 à 3, dans lequel :

les moyens de stockage d'informations biomédicales (11a), les moyens de détermination d'exécution de simulation (11b) et les moyens de prise de décision d'opération (11c) sont installés dans un établissement médical où l'opération est effectuée sur le patient,

les moyens de génération de modèle (12a) et les moyens d'exécution de simulation (12b) sont installés dans un centre de simulation, et

les moyens de détermination du taux d'amélioration (12c) et les moyens de remboursement de frais de traitement (13a) sont installés dans une compagnie d'assurance maladie pour rembourser les coûts de traitement médical.

5. Système de simulation biomédicale selon l'une quelconque des revendications 1 à 4, dans lequel :

les moyens de stockage d'informations biomédicales (11a) et les moyens de détermination d'exécution de simulation (11b) sont installés dans un établissement médical où l'opération est effectuée sur le patient,

les moyens de génération de modèle (12a) et les moyens d'exécution de simulation (12b) sont installés dans un centre de simulation, et

les moyens de détermination de taux d'amélioration (12c), les moyens de prise de décision d'opération (11c) et les moyens de remboursement de frais de traitement (13a) sont installés dans une compagnie d'assurance maladie pour rembourser les coûts de traitement médical.

6. Système de simulation biomédicale selon l'une quelconque des revendications 1 à 5, dans lequel :

les moyens de prise de décision d'opération (11c) comprennent un premier moyen de prise de décision pour délivrer en sortie une demande de remboursement d'assurance lorsque les moyens de détermination de taux d'amélioration (12c) ont déterminé que les résultats de la simulation biomédicale satisfont au taux d'amélioration prédéterminé, et un second moyen de prise de décision pour décider d'effectuer l'opération sur le patient lorsque les moyens de détermination de taux d'amélioration (12c) ont déterminé que les résultats de la simulation biomédicale satisfont au taux d'amélioration prédéterminé et, ensuite, la première unité de prise de décision a délivré en sortie l'application de remboursement d'assurance,

les moyens de stockage d'informations biomédicales (11a), les moyens de détermination d'exécution de simulation (11b) et le premier moyen de prise de décision sont installés dans un établissement médical où l'opération est effectuée sur le patient,

les moyens de génération de modèle (12a), les moyens d'exécution de simulation (12b) et les moyens de détermination de taux d'amélioration (12c) sont installés dans un centre de simulation, et

le second moyen de prise de décision et les moyens de remboursement de frais de traitement (13a) sont installés dans une compagnie d'assurance maladie pour rembourser les coûts de traitement médical.

7. Système de simulation biomédicale selon l'une quelconque des revendications 1 à 6, dans lequel :

les moyens de remboursement de frais de traitement (13a) déterminent de rembourser les coûts de traitement médical lorsque les moyens de détermination de taux d'amélioration (12c) ont déterminé que les résultats de la simulation biomédicale satisfont au taux d'amélioration prédéterminé, et

les moyens de prise de décision d'opération (11c) décident d'effectuer l'opération sur le patient lorsque les moyens de détermination de taux d'amélioration (12c) ont déterminé que les résultats de la simulation biomédicale satisfont le taux d'amélioration prédéterminé et, ensuite, les moyens de prise de décision d'opération (11c) obtiennent la confirmation des moyen de remboursement de frais de traitement (13a) ayant décidé de rembourser les coûts de traitement médical.

8. Système de simulation biomédicale selon l'une quelconque des revendications 1 à 7, dans lequel :
les moyens de remboursement de frais de traitement (13a) remboursent les coûts de traitement médical et les coûts d'exécution de la simulation biomédicale lorsque l'opération est réalisée sur le patient, et remboursent les frais d'exécution de la simulation biomédicale lorsque l'opération n'est pas effectuée sur le patient.

9. Système de simulation biomédicale selon l'une quelconque des revendications 1 à 8, comprenant en outre :

des moyens de stockage de données de caractéristiques de dispositif (280) pour stocker des données de caractéristiques de dispositif indiquant des caractéristiques du dispositif de thérapie de resynchronisation cardiaque à utiliser lors de l'opération du patient,

dans lequel les moyens d'exécution de simulation (12b) font référence aux données de caractéristiques de dispositif pour exécuter la simulation biomédicale dans laquelle sont reflétées les caractéristiques du dispositif de thérapie de resynchronisation cardiaque.

10. Système de simulation biomédicale selon l'une quelconque des revendications 1 à 9, dans lequel :
les moyens de détermination de taux d'amélioration (12c) sélectionnent, parmi les procédures opératoires applicables, une procédure opératoire avec les résultats de la simulation biomédicale montrant un taux d'amélioration le plus élevé, et déterminent si le taux d'amélioration le plus élevé répond au taux d'amélioration prédéterminé.

11. Système de simulation biomédicale selon l'une quelconque des revendications 1 à 10, dans lequel :
les moyens de détermination de taux d'amélioration (12c) effectuent la détermination associée au taux d'amélioration prédéterminé sur la base d'une valeur d'évaluation qui est une somme de valeurs, chacune des valeurs étant obtenue en multipliant un taux d'amélioration de chacun d'une pluralité d'indices, obtenus pour chaque motif de disposition d'électrodes, par une valeur de pondération correspondant à l'indice, le taux d'amélioration de chacun des indices étant inclus dans les résultats obtenus à partir de la simulation biomédicale exécutée pour les procédures opératoires individuelles.

12. Système de simulation biomédicale selon revendication 11, dans lequel :
la valeur de pondération correspondant à chacun des indices est fixée à l'avance d'un point de vue médical.

13. Procédé de simulation biomédicale exécuté par un système de simulation biomédicale comprenant au moins un ordinateur, le procédé de simulation biomédicale comprenant les étapes consistant à :

déterminer, sur la base d'informations biomédicales spécifiques à un patient, s'il faut ou non effectuer une simulation biomédicale ;

générer un modèle biomédical d'un cœur du patient sur la base des informations biomédicales lorsqu'il a déterminé d'effectuer la simulation biomédicale ;

exécuter, sur la base du modèle biomédical, la simulation biomédicale de conditions postopératoires du cœur après une opération d'implantation d'un dispositif de thérapie de resynchronisation cardiaque effectuée sur le patient pour chacun d'une pluralité de motifs de disposition d'électrodes associés à l'implantation dispositif de thérapie de resynchronisation cardiaque ;

déterminer si des résultats de la simulation biomédicale satisfont ou non à un taux d'amélioration prédéterminé associé aux symptômes ;

déterminer, lorsque les résultats de la simulation biomédicale ne satisfont pas au taux d'amélioration prédéterminé, de ne pas rembourser les coûts de traitement médical pour l'opération du patient ; et

décider d'effectuer l'opération sur le patient lorsque les résultats de la simulation biomédicale satisfont le taux d'amélioration prédéterminé.

**14.** Programme informatique qui, lorsqu'il est exécuté par un système de simulation biomédicale incluant au moins un ordinateur, amène le système de simulation biomédicale à exécuter le procédé selon la revendication 13.

FIG. 1

FIG. 2

FIG. 3

FIG. 4

HOSPITAL
100 INFORMATION
SYSTEM

101 PROCESSOR

104 GRAPHICS
PROCESSING
UNIT

21 MONITOR

22 KEYBOARD

102 MEMORY

105 INPUT
INTERFACE

23 MOUSE

103 HDD

106 OPTICAL
DRIVE UNIT

24 OPTICAL DISK

108 NETWORK
INTERFACE

107 DEVICE
CONNECTION
INTERFACE

25 MEMORY DEVICE

109 BUS

26 MEMORY
READER/WRITER

27 MEMORY
CARD

20 NETWORK

FIG. 5

FIG. 6

FIG. 7

100 HOSPITAL INFORMATION SYSTEM

110 PATIENT DATA STORING UNIT

PATIENT DATA SET
·MRI/CT
·ECG DATA
·STROKE VOLUME
·EJECTION FRACTION
·BLOOD PRESSURE dP/dt
·INFARCTION AREA DATA

S121 SIMULATION REQUEST (WITH PATIENT DATA)

S132 DETERMINATION BY DOCTOR ON IMPLEMENTATION OF CRT TREATMENT (NO INSURANCE REIMBURSEMENT)

S131 DECISION X+Y
0
X=1
1
Y=0

S133 IMPLEMENTATION OF CRT TREATMENT BASED ON SIMULATION RESULTS (INSURANCE REIMBURSEMENT AVAILABLE)

300

S130 NOTIFICATION OF INSURANCE COVERAGE BEING NOT AVAILABLE
MEDICAL EXPENSE REIMBURSEMENT SYSTEM

200 CRT SIMULATION SYSTEM

230 PATIENT DATA STORING UNIT
MRI/CT
STROKE VOLUME EJECTION FRACTION BLOOD PRESSURE
INFARCTION AREA
ECG DATA
BLOOD PRESSURE dP/dt

S122 CREATION OF CARDIAC MORPHOLOGY MODEL (PREPARATION STEP)

S123 DETERMINATION OF PARAMETERS FOR DYNAMIC SYSTEM SIMULATION

S124 DETERMINATION OF PARAMETERS FOR ELECTRICAL SYSTEM SIMULATION

S125 DESIGNATION OF (N) ELECTRODE DISPOSITION PATTERNS

S126 EXECUTION OF HEART SIMULATION

210 CRT DEVICE LIST STORING UNIT

S127 SORTING OF VALUES OF EACH INDEX (EF AND (dP/dt)$_{max}$) IN DESCENDING ORDER

S128 IMPROVEMENT RATE OF EF OR (dP/dt)$_{max}$ ≥ SPECIFIED VALUE?
YES
NO

S129 TRANSMISSION OF INFORMATION ON OPTIMAL CRT DEVICE AND ELECTRODE LOCATIONS

DYNAMIC SYSTEM
SIMULATION PARAMETER
DETERMINATION

( START )

TUNE PARAMETERS (STROKE
VOLUME, BLOOD PRESSURE,
AND INFARCTION AREA) — S141

HEART SIMULATION — S142

CHECK PREOPERATIVE
STATUS — S143

DISPLAY HEART BEHAVIOR — S144

( END )

FIG. 8

HEART SIMULATION

START

$t = t + \Delta t$ — S151

PERFORM FSI ANALYSIS — S152

UPDATE ALE MESH — S153

HAS SIMULATION CONVERGED? — S154

NO

YES

$t = t_{end}$? — S155

NO

YES

RETURN

FIG. 9

FIG. 10

PARAMETER TUNING

```
          START
            │
            ▼
    ┌─────────────────┐   S181
    │      i=1        │
    └─────────────────┘
            │
            ▼
    ┌─────────────────┐   S182
    │ DETERMINE INPUT │
    │  PARAMETER (xᵢ) │
    └─────────────────┘
            │
            ▼
    ┌─────────────────┐   S183
    │    POTENTIAL    │
    │ CALCULATION (xᵢ)│
    └─────────────────┘
            │
            ▼
    ┌─────────────────┐   S184
    │ ACQUIRE ECG (fᵢ(t)) │
    └─────────────────┘
            │
            ▼
    ┌─────────────────┐   S185
    │ STORE xᵢ AND (fᵢ(t)) │
    └─────────────────┘
            │
            ▼
 NO      ◇─────────◇    S186
◄────────│ i=iₘₐₓ ? │
         ◇─────────◇
   │          │ YES
   │          ▼
┌─────────┐ ┌──────────────────┐
│  i=i+1  │ │ SELECT xᵢ WITH HIGHEST │
└─────────┘ │ CROSS CORRELATION │
 S187       └──────────────────┘
              S188
                │
                ▼
            RETURN
```

$i=1$ — S181

DETERMINE INPUT PARAMETER $(x_i)$ — S182

POTENTIAL CALCULATION $(x_i)$ — S183

ACQUIRE ECG $(f_i(t))$ — S184

STORE $x_i$ AND $(f_i(t))$ — S185

$i=i_{max}$ ? — S186

$i=i+1$ — S187

SELECT $x_i$ WITH HIGHEST CROSS CORRELATION — S188

FIG. 11

POTENTIAL CALCULATION

```
              ┌──────────┐
              │  START   │
              └──────────┘
                   │
                   ▼
     ┌─────────────────────────────────┐  S191
     │ INPUT CARDIAC MORPHOLOGY MODEL AND
     │ PARAMETER USED TO MAKE SIMULATED ECG
     │     RESEMBLE MEASURED ECG         │
     └─────────────────────────────────┘
                   │
                   ▼
     ┌─────────────────────────────────┐  S192
     │          t = t + Δt              │
     └─────────────────────────────────┘
                   │
                   ▼
     ┌─────────────────────────────────┐  S193
     │ CALCULATE TIME EVOLUTION OF MEMBRANE
     │ POTENTIAL Vm ACCORDING TO CELL MODEL │
     └─────────────────────────────────┘
                   │
                   ▼
     ┌─────────────────────────────────┐  S194
     │ DISCRETIZE BI-DOMAIN EQUATION USING
     │ FINITE ELEMENT METHOD TO OBTAIN SYSTEM
     │ OF LINEAR EQUATIONS FOR EXTRACELLULAR
     │         POTENTIAL φe             │
     └─────────────────────────────────┘
                   │
                   ▼
     ┌─────────────────────────────────┐  S195
     │   SOLVE SYSTEM OF LINEAR EQUATIONS │
     └─────────────────────────────────┘
                   │
                   ▼
     ┌─────────────────────────────────┐  S196
     │ CALCULATE VALUES OF DEPENDENT
     │        VARIABLES (INTERCELLULAR
     │ (EXTRACELLULAR) POTENTIAL φi(φe)) FOR
     │      DISCRETE NEXT TIME STEP      │
     └─────────────────────────────────┘
                   │
                   ▼
     ┌─────────────────────────────────┐  S197
     │            OUTPUT                │
     └─────────────────────────────────┘
                   │
                   ▼
              ◇ S198
         ◇ t = tend ? ◇ ──── NO
              ◇
             YES
              │
              ▼
         ┌──────────┐
         │  RETURN  │
         └──────────┘
```

**FIG. 12**

FIG. 13

HEART SIMULATION
(BIVENTRICULAR PACING)

START

DETERMINE 1ST RIGHT-VENTRICLE
ELECTRODE DISPOSITION LOCATION
(AMONGST Na) — S211

DETERMINE 1ST LEFT-VENTRICLE
ELECTRODE DISPOSITION LOCATION
(AMONGST Nb) — S212

RUN HEART SIMULATION OF CRT DEVICE
PERFORMING BIVENTRICULAR PACING — S213

CALCULATE EJECTION FRACTION AND
$(dP/dt)_{max}$ — S214

IS THERE UNSELECTED LEFT-
VENTRICLE ELECTRODE DISPOSITION
ALLOWABLE LOCATION? — S215

YES

NO

IS THERE UNSELECTED RIGHT-
VENTRICLE ELECTRODE DISPOSITION
ALLOWABLE LOCATION? — S216

YES

NO

RETURN

FIG. 14

HEART SIMULATION
(TRIVENTRICULAR PACING)

START

DETERMINE 1ST RIGHT-VENTRICLE
ELECTRODE DISPOSITION LOCATION
(AMONGST Na) — S221

DETERMINE 1ST LEFT-VENTRICLE
ELECTRODE DISPOSITION LOCATION
(AMONGST Nb) — S222

DETERMINE 2ND LEFT-VENTRICLE
ELECTRODE DISPOSITION LOCATION
(AMONGST Nb) — S223

RUN HEART SIMULATION OF CRT DEVICE
PERFORMING TRIVENTRICULAR PACING — S224

CALCULATE EJECTION FRACTION AND
$(dP/dt)_{max}$ — S225

IS THERE UNSELECTED LEFT-
VENTRICLE ELECTRODE LOCATION
PAIR? — S226

YES

NO

IS THERE UNSELECTED RIGHT-
VENTRICLE ELECTRODE DISPOSITION
ALLOWABLE LOCATION? — S227

YES

NO

RETURN

FIG. 15

EP 3 125 141 B1

HEART SIMULATION
(QUADRIVENTRICULAR PACING)

START

DETERMINE 1ST RIGHT-VENTRICLE
ELECTRODE DISPOSITION LOCATION
(AMONGST Na) — S231

DETERMINE 2ND RIGHT-VENTRICLE
ELECTRODE DISPOSITION LOCATION
(AMONGST Na) — S232

DETERMINE 1ST LEFT-VENTRICLE
ELECTRODE DISPOSITION LOCATION
(AMONGST Nb) — S233

DETERMINE 2ND LEFT-VENTRICLE
ELECTRODE DISPOSITION LOCATION
(AMONGST Nb) — S234

RUN HEART SIMULATION OF CRT DEVICE
PERFORMING QUADRIVENTRICULAR
PACING — S235

CALCULATE EJECTION FRACTION AND
$(dP/dt)_{max}$ — S236

IS THERE UNSELECTED LEFT-VENTRICLE
ELECTRODE LOCATION PAIR? — S237

YES

NO

IS THERE UNSELECTED RIGHT-VENTRICLE
ELECTRODE LOCATION PAIR? — S238

YES

NO

RETURN

FIG. 16

DETERMINATION OF OPTIMAL CRT
DEVICE AND ELECTRODE LOCATIONS

START

S241

i = 1

S242

CALCULATE $\alpha_{i,EF} = EF_i / EF_e$

S243

CALCULATE $\alpha_{i,p} = (dP_i/dt)_{max}/(dP_e/dt)_{max}$

S244

CALCULATE $\alpha_{i,LVESV} = LVESV_i / LVESV_e$

S245

CALCULATE $\alpha_{i,SV} = SV_i / SV_e$

S246

CALCULATE $\alpha_{i,MR} = MR_i / MR_e$

S247

CALCULATE $\alpha_{i,PP} = PP_i / PP_e$

S248

CALCULATE $\alpha_{i,IVA} = IVA_i / IVA_e$

S249

CALCULATE $\alpha_{i,ET} = ET_i / ET_e$

S250

CALCULATE OPTIMIZATION EVALUATION VALUE $F_i$
$= F(\alpha_{i,EF}, \alpha_{i,p}, \alpha_{i,LVESV}, \alpha_{i,SV}, \alpha_{i,MR}, \alpha_{i,PP}, \alpha_{i,IVA}, \alpha_{i,ET})$

S251

$i \geq N$ ?

NO

$i = i + 1$   S252

YES

S253

DETERMINE OPTIMAL CRT DEVICE AND ELECTRODE
LOCATIONS BASED ON ELECTRODE DISPOSITION PATTERN
YIELDING MAXIMUM VALUE OF $F_i$

RETURN

FIG. 17

FIG. 18

31

HOSPITAL

100

HOSPITAL
INFORMATION
SYSTEM

S301

PATIENT DATA

32

HSC

200

CRT
SIMULATION
SYSTEM

S302

SIMULATION RESULTS

S303
DETERMINATION
RESULT

33

300

DETERMINATION ON
WHETHER TO MAKE
INSURANCE
REIMBURSEMENT

MEDICAL EXPENSE
REIMBURSEMENT
SYSTEM

HEALTHCARE
INSURANCE COMPANY

FIG. 19

FIG. 20

# FIG. 21

FIG. 22

FIG. 23

FIG. 24

FIG. 25

# FIG. 26

**FIG. 27**

# FIG. 28

100 — HOSPITAL INFORMATION SYSTEM

PATIENT DATA STORING UNIT — 110

PATIENT DATA SET
·MRI/CT
·ECG DATA
·STROKE VOLUME
·EJECTION FRACTION
·BLOOD PRESSURE
dP/dt
·INFARCTION AREA DATA

SIMULATION REQUEST (WITH PATIENT DATA) — S121

DETERMINATION BY DOCTOR ON IMPLEMENTATION OF CRT TREATMENT (NO INSURANCE REIMBURSEMENT) — S132

DECISION X + Y — S131
X=1
0
1
Y=0

IMPLEMENTATION OF CRT TREATMENT BASED ON SIMULATION RESULTS (INSURANCE REIMBURSEMENT AVAILABLE) — S133

300 — NOTIFICATION OF INSURANCE COVERAGE BEING NOT AVAILABLE — S130

MEDICAL EXPENSE REIMBURSEMENT SYSTEM

CRT SIMULATION SYSTEM — 200

PATIENT DATA STORING UNIT — 230

CREATION OF CARDIAC MORPHOLOGY MODEL (PREPARATION STEP) — S122

DETERMINATION OF PARAMETERS FOR DYNAMIC SYSTEM SIMULATION — S123

DETERMINATION OF PARAMETERS FOR ELECTRICAL SYSTEM SIMULATION — S124

DESIGNATION OF (N) ELECTRODE DISPOSITION PATTERNS — S125

DEVICE CHARACTERISTICS DATA STORING UNIT — 280

EXECUTION OF HEART SIMULATION — S126

CRT DEVICE LIST STORING UNIT — 210

TRANSMISSION OF INFORMATION ON OPTIMAL CRT DEVICE AND ELECTRODE LOCATIONS — S129

IMPROVEMENT RATE OF EF OR (dP/dt)$_{max}$ ≥ SPECIFIED VALUE? — S128
YES
NO

SORTING OF VALUES OF EACH INDEX (EF AND (dP/dt)$_{max}$) IN DESCENDING ORDER — S127

MANUFACTURER INFORMATION SYSTEM — 400

DEVICE SPECIFICATION PROVIDING UNIT — 410

61

FIG. 29

FIG. 30

FIG. 31

FIG. 32

FIG. 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011098175 A
- JP 2008534165 A
- JP 2010528683 A
- US 20130197881 A1

### Non-patent literature cited in the description

- **JASON CONSTANTINO ; YUXUAN HU ; NATALIA A. TRAYANOVA.** A computational approach to understanding the cardiac electromechanical activation sequence in the normal and failing heart, with translation to the clinical practice of CRT. *Progress in Biophysics and Molecular Biology,* October 2012, vol. 110, 372-379

- **SERMESANT M ; CHABINIOK R ; CHINCHAPATNAM P ; MANSI T ; BILLET F ; MOIREAU P ; PEYRAT JM ; WONG K ; RELAN J ; RHODE K.** Patient-specific electromechanical models of the heart for the prediction of pacing acute effects in CRT: A preliminary clinical validation. *Medical Image Analysis,* January 2012, vol. 16 (1), 201-215
- **OKADA J ; SASAKI T ; WASHIO T ; YAMASHITA H ; KARIYA T ; IMAI Y ; NAKAGAWA M ; KADOOKA Y ; NAGAI R ; HISADA T.** Patient Specific Simulation of Body Surface ECG using the Finite Element Method. *Pacing and Clinical Electrophysiology,* March 2013, vol. 36 (3), 309-321